(19) Europäisches Patentamt
European Patent Office
Office européen des brevets

(11) EP 4 674 438 A1

(12) EUROPEAN PATENT APPLICATION
published in accordance with Art. 153(4) EPC

(43) Date of publication:
**07.01.2026 Bulletin 2026/02**

(21) Application number: **24764047.7**

(22) Date of filing: **01.03.2024**

(51) International Patent Classification (IPC):
***A61K 47/68*** $^{(2017.01)}$ ***A61K 38/08*** $^{(2019.01)}$
***A61K 39/395*** $^{(2006.01)}$ ***A61P 35/00*** $^{(2006.01)}$
***A61P 43/00*** $^{(2006.01)}$

(52) Cooperative Patent Classification (CPC):
**A61K 38/08; A61K 39/395; A61K 47/68; A61P 35/00; A61P 43/00**

(86) International application number:
**PCT/JP2024/007871**

(87) International publication number:
**WO 2024/181570 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.03.2023 JP 2023032088**

(71) Applicant: **Taiho Pharmaceutical Co., Ltd.**
**Chiyoda-ku**
**Tokyo 101-8444 (JP)**

(72) Inventors:
- **YOSHIMURA Chihoko**
  **Tsukuba-shi, Ibaraki 300-2611 (JP)**
- **OSADA Akiko**
  **Tsukuba-shi, Ibaraki 300-2611 (JP)**
- **HIRANO Atsushi**
  **Tsukuba-shi, Ibaraki 300-2611 (JP)**

(74) Representative: **Wächtershäuser & Hartz Patentanwaltspartnerschaft mbB**
**Weinstraße 8**
**80333 München (DE)**

Remarks:
The complete document including Reference Table(s) and the Sequence Listing(s) can be downloaded from the EPO website

# (54) ANTIBODY-DRUG CONJUGATE CONTAINING ANTI-CD138 ANTIBODY

(57) This invention provides an antibody-drug conjugate (ADC) that binds specifically to CD138 and exerts excellent anti-tumor effects thereon. Such antibody-drug conjugate (ADC) comprises the anti-CD138 antibody comprising a particular amino acid sequence and monomethyl auristatin E bound thereto via a linker.

EP 4 674 438 A1

Fig. 5

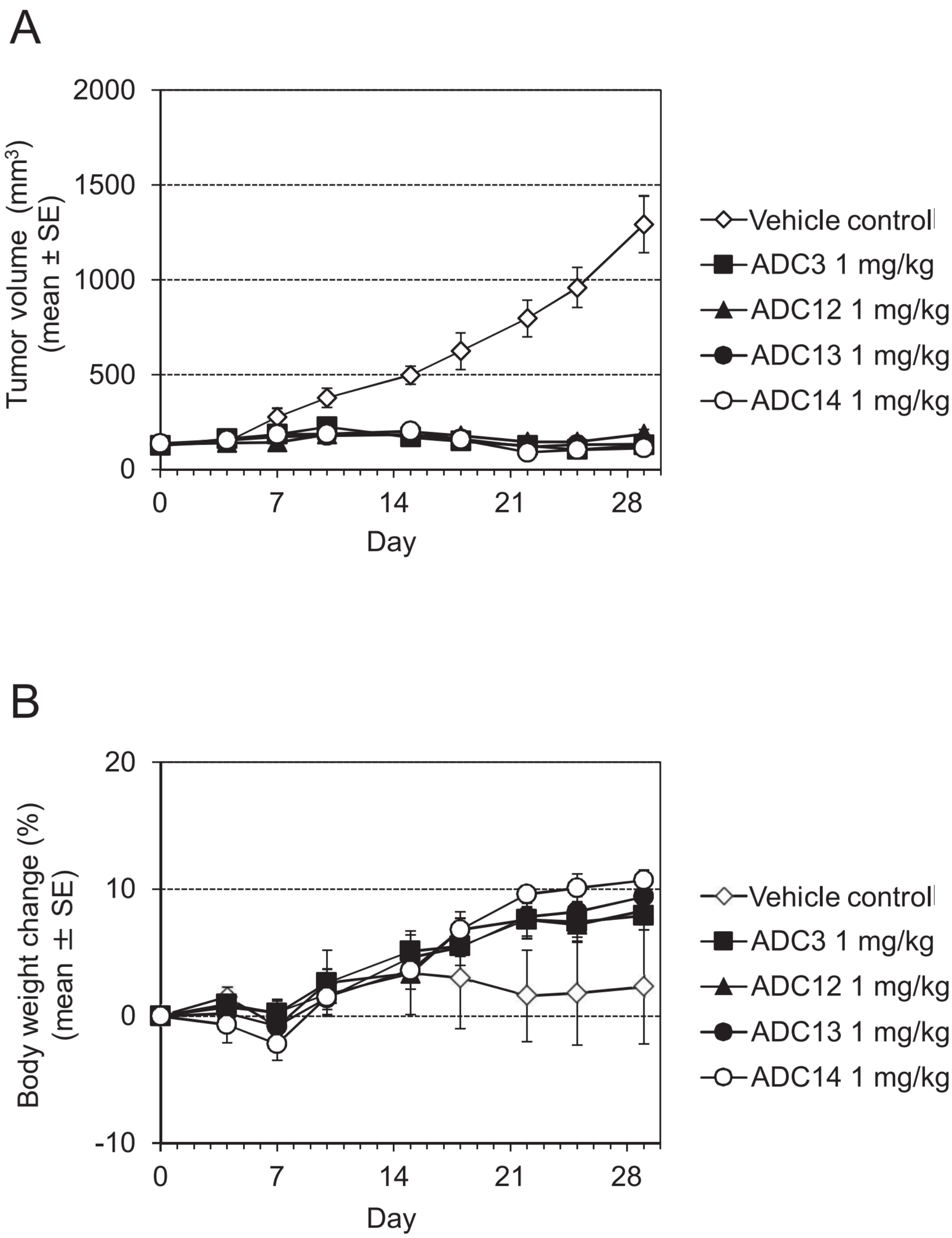
A
2000
1500
1000
500
0
Tumor volume (mm3)
(mean ± SE)
0
7
14
21
28
Day
Vehicle controll
ADC3 1 mg/kg
ADC12 1 mg/kg
ADC13 1 mg/kg
ADC14 1 mg/kg
B
20
10
0
-10
Body weight change (%)
(mean ± SE)
0
7
14
21
28
Day
Vehicle controll
ADC3 1 mg/kg
ADC12 1 mg/kg
ADC13 1 mg/kg
ADC14 1 mg/kg

## Description

Technical Field

The present invention relates to an antibody-drug conjugate (ADC) comprising a drug bound to an antibody that specifically binds to CD138 via a linker and an antibody that can be used for such antibody-drug conjugate.

Background Art

An antibody-drug conjugate (ADC) comprising a cytotoxic drug bound to an antibody that binds to an antigen expressed on a cancer cell surface and internalized in a cell can deliver a drug selectively to a cancer cell. Accordingly, ADC is expected to accumulate a drug in a cancer cell and kill the cancer cell (Non Patent Literature 1 to Non Patent Literature 3). As ADC, for example, Mylotarg (registered trademark: Gemtuzumab ozogamicin) comprising the anti-CD33 antibody and Calicheamicin bound thereto has been approved as a therapeutic agent for acute myelocytic leukemia. Also, Adcetris (registered trademark: Brentuximab vedotin) comprising the anti-CD30 antibody and monomethyl auristatin E (MMAE) bound thereto has been approved as a therapeutic agent for Hodgkin's lymphoma and anaplastic large cell lymphoma (Non Patent Literature 4). Concerning the targets of ADCs that have been approved in the past, a large number of target molecules are known to be expressed on the cancer cell surface.

CD138 functions as an extracellular matrix receptor, and CD138 has been reported to be expressed in multiple myeloma (MM) cells and tissue of, for example, pancreatic cancer, breast cancer, prostate cancer, or non-Hodgkin's lymphoma (Non Patent Literature 5 and Non Patent Literature 6). In recent years, the dynamics of CD138 (CD138 internalization and recycling) has drawn attention (Non Patent Literature 7). Such properties may contribute to active intracellular uptake of ADC targets and CD138 is thus preferable as an ADC target.

An example of a CD138-targeting ADC is BT062 comprising the Maytansine analog DM4 bound to lysine of the chimeric IgG4 antibody nBT062 via an SPDB linker (Indatuximab ravatansine) (Patent Literature 1). BT062 binds to CD138 expressed on the multiple myeloma cell surface and exerts high cytotoxic activity and anti-tumor effects (Non Patent Literature 8). While BT062 have been subjected to clinical trials, the effects thereof are limited, and, accordingly, development thereof has been suspended. In clinical trials of BT062, in addition, disappearance of BT062 from blood at a relatively early stage, ocular toxicity presumed to be derived from DM4, and expression of the anti-antibody in the patient's blood have been reported (Non Patent Literature 9).

While an immunogenicity risk is generally increased in order from a complete human antibody, a humanized antibody, a chimeric antibody, to a heterologous antibody (e.g., a mouse antibody), a chimeric antibody is used as an antibody component of BT062. Accordingly, an immunogenicity risk is an issue of concern.

While a chimeric IgG1 antibody class-changed from nBTO62 has been known (Patent Literature 2), this antibody is used as a fusion protein comprising a masked interferon bound thereto, and it is not used as an antibody component of ADC.

In the development of a novel antibody-drug conjugate that can satisfy the clinical needs, it is necessary to combine various types of antibodies, linkers, and payloads. However, a small difference in antibody sequences could change physical properties and cause antibody aggregation, changes in the linker-payload reactivity, ADC aggregation, and lowered stability. Accordingly, there is no general-purpose linker-payload combination that is applicable to any antibody, and it is necessary to screen for every antibody sequence and discover an adequate combination. Therefore, it is the general technical knowledge in the art that discovery of an antibody-drug conjugate useful as a pharmaceutical preparation would not be easy for a person skilled in the art. Examples of payloads that have been approved for ADCs include MMAE, Dxd, and DM1, although no ADCs comprising such payloads bound to the CD138 antibody have been known.

As described above, the necessity of therapeutic agents for cancer expressing CD138, which is expressed at high levels in various tumor cells, has been recognized, and CD138 is considered as a potential ADC target because its expression on a cell surface and its internalizing capability. At present, however, no ADCs that have overcome the problem as described above are known.

Citation List

Patent Literature

Patent Literature 1: WO 2009/080830
Patent Literature 2: WO 2020/214690

Non Patent Literature

Non Patent Literature 1: Bioconjugate Chem., 2010, 21, 5-13
Non Patent Literature 2: Current Opinion in Chemical Biology, 2010, 14, 529-537
Non Patent Literature 3: Expert Opin. Biol. Ther., 2004, 4, 1445-1452
Non Patent Literature 4: Nature Biotechnology, 2012, 30, 631-637
Non Patent Literature 5: Oncotarget, 2015, 6 (30): 28693-28715
Non Patent Literature 6: Am. J. Physiol. Cell Physiol., Jul 1, 2022; 323 (1): C29-C45
Non Patent Literature 7: Nature, 2019, 568 (7752): 410-414
Non Patent Literature 8: J. Hematol. Oncol., 2017, 11: 10 (1): 13
Non Patent Literature 9: Clin. Lymphoma Myeloma Leuk., 2019, 19 (6): 372-380

Summary of Invention

Technical Problem

The problem to be solved of the present invention is to provide an antibody-drug conjugate that binds specifically to a CD138-expressing cancer cell and exerts excellent anti-tumor effects. In addition, the problem to be solved of the present invention is to provide an antibody that is suitable as an antibody component of an antibody-drug conjugate that is useful as an anti-tumor agent.

Solution to Problem

The present inventors have conducted concentrated studies. As a result, they discovered a sequence of a humanized antibody with the IgG1 constant region and maintaining the binding capability specifically to CD138. They further discovered an antibody-drug conjugate that would not aggregate in a solution, that would exhibit excellent stability in plasma, and that would exert high anti-tumor effects by using the humanized antibody in combination with various linkers and payloads. This has led to the completion of the present invention. The ADC comprising a humanized IgG1 antibody according to the present invention is expected to be more stabilized and have reduced immunogenic potentials *in vivo.* In addition, ADC exhibiting excellent stability in plasma would reduce a risk of the onset of toxicity caused by drug dissociation in blood. While denaturation or aggregation of antibodies may lower drug efficacy or cause unexpected side effects, the ADC of the present invention is less likely to cause denaturation or aggregation upon heat application and exerts excellent stability as a pharmaceutical preparation.
The present invention has the following features.

[1] An antibody-drug conjugate (ADC) represented by Formula 1:

$$\text{Ab-(L-D)p} \qquad (1)$$

wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents a linker;
D represents monomethyl auristatin; and

p is 1 to 12.

[2] The antibody-drug conjugate (ADC) according to [1], wherein Ab represents an antibody selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138.

[3] The antibody-drug conjugate (ADC) according to [1] or [2], wherein Ab represents an antibody selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34.

[4] The antibody-drug conjugate (ADC) according to any one of [1] to [3], wherein L represents 6-maleimidocaproyl-valine-citrulline-p-aminobenzyloxycarbonyl (MC-Val-Cit-PAB).
[5] The antibody-drug conjugate (ADC) according to any one of [1] to [4], wherein D represents monomethyl auristatin E.
[6] The antibody-drug conjugate (ADC) according to any one of [1] to [5], wherein p is 1 to 8.
[7] The antibody-drug conjugate (ADC) according to any one of [1] to [6],
wherein

Ab represents an antibody comprising a heavy chain comprising a variable region consisting of the amino acid sequence represented by SEQ ID NO: 14 and a light chain comprising a variable region consisting of the amino acid sequence represented by SEQ ID NO: 16,
L represents 6-maleimidocaproyl-valine-citrulline-p-aminobenzyloxycarbonyl (MC-Val-Cit-PAB),
D represents monomethyl auristatin E, and
P is 1 to 8.

[8] The antibody-drug conjugate (ADC) according to any one of [1] to [7],
wherein

Ab represents an antibody comprising a heavy chain consisting of the amino acid sequence represented by SEQ ID NO: 44 and a light chain consisting of the amino acid sequence represented by SEQ ID NO: 34,
L represents 6-maleimidocaproyl-valine-citrulline-p-aminobenzyloxycarbonyl (MC-Val-Cit-PAB),

D represents monomethyl auristatin E, and
P is 1 to 8.

[9] A pharmaceutical composition comprising the antibody-drug conjugate (ADC) according to any one of [1] to [8] and a carrier.
[10] An anti-tumor agent comprising the antibody-drug conjugate (ADC) according to any one of [1] to [8].
[11] The anti-tumor agent according to [10], which is used for treating cancer that expresses CD138.
[12] The anti-tumor agent according to [10], which is administered in combination with another anti-tumor agent.
[13] The anti-tumor agent according to [10], which is used for treatment of gastric cancer, pancreatic cancer, large bowel cancer, lung cancer, breast cancer, or urothelial cancer.
[14] An antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16.

[15] The antibody according to [14], which is selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138.

[16] The antibody according to [14] or [15], which is selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34.

[17] A method for treating cancer comprising a step of administering the antibody-drug conjugate (ADC) according to any one of [1] to [8] to a subject.

[18] Use of the antibody-drug conjugate (ADC) according to any one of [1] to [8] for treating cancer.
[19] The antibody-drug conjugate (ADC) according to any one of [1] to [8], which is used for treating cancer.
[20] Use of the antibody-drug conjugate (ADC) according to any one of [1] to [8] f in the manufacture of an anti-tumor agent.
[21] A method for treating cancer comprising a step of administering the antibody-drug conjugate (ADC) according to any one of [1] to [8] and another anti-tumor agent to a subject.
[22] Use of the antibody-drug conjugate (ADC) according to any one of [1] to [8] for treating cancer, which is administered in combination with another anti-tumor agent.
[23] The antibody-drug conjugate (ADC) according to any one of [1] to [8] used for treating cancer, which is administered in combination with another anti-tumor agent.
[24] Use of the antibody-drug conjugate (ADC) according to any one of [1] to [8] in the manufacture of an anti-tumor agent, which is administered in combination with another anti-tumor agent.
[25] The antibody-drug conjugate (ADC) according to any one of [1] to [8], which has stable physical properties.
[26] The antibody-drug conjugate (ADC) according to any one of [1] to [8], wherein the denaturation midpoint temperature $T_m2$ is 80°C or higher and the aggregation initiation temperature $T_{agg}266$ is 70°C or higher.

The description incorporates the contents disclosed by JP Patent Application No. 2023-032088, based on which the priority of the present application claims.

Advantageous Effects of Invention

The antibody-drug conjugate (ADC) of the present invention has the excellent binding capability. It thus binds specifically to a cancer cell expressing CD138 and is internalized in the cancer cell. Thus, the ADC of the present invention exerts excellent anti-tumor effects. In addition, the ADC of the present invention exhibits excellent stability in plasma, and, accordingly, it is less likely to have toxicity due to drug dissociation in blood.
Also, the antibody of the present invention has an excellent CD138-binding capability and is useful as a reagent to detect CD138. In addition, the antibody of the present invention is useful as an antibody component of ADC.
In addition, the antibody and the ADC of the present invention are less likely to be aggregated, and, accordingly, the antibody and the ADC of the present invention are useful as pharmaceutical preparations and starting materials therefor.

Brief Description of Drawings

[Figure 1] Figure 1 shows the binding capability of Antibody 1 and of Antibody 3 to Antibody 9 to AsPC-1 cells.

[Figure 2-1] Figure 2-1 shows combinations of drug linkers bound to antibodies of ADCs obtained with the use of the anti-CD138 antibody (ADC1 to ADC5).

[Figure 2-2] Figure 2-2 shows combinations of drug linkers bound to antibodies of ADCs obtained with the use of the anti-CD138 antibody (ADC6 to ADC10, continued from Figure 2-1).

[Figure 2-3] Figure 2-3 shows combinations of drug linkers bound to antibodies of ADCs obtained with the use of the anti-CD138 antibody (ADC11 to ADC14, continued from Figure 2-2).

[Figure 3] Figure 3 shows the binding capability of Antibody 1 to Capan-1 cells (Figure 3 A) and the binding incapacity thereof to Jurkat cells (Figure 3 B).

[Figure 4] Figure 4 shows anti-tumor effects of ADC2 and ADC3 *(in vivo).* Figure 4 A shows changes in tumor volume with the elapse of time and Figure 4 B shows changes in body weight with the elapse of time.

[Figure 5] Figure 5 shows anti-tumor effects of ADC3 and ADC12 to ADC14 *(in vivo)*. Figure 5 A shows changes in tumor volume with the elapse of time and Figure 5 B shows changes in body weight with the elapse of time.

[Figure 6] Figure 6 shows sequences of SEQ ID NOs: 1 to 7.

[Figure 7] Figure 7 shows sequences of SEQ ID NOs: 8 to 12.

[Figure 8] Figure 8 shows sequences of SEQ ID NOs: 13 to 17.

[Figure 9] Figure 9 shows sequences of SEQ ID NOs: 18 to 22.

[Figure 10] Figure 10 shows sequences of SEQ ID NOs: 23 to 25.

[Figure 11] Figure 11 shows sequences of SEQ ID NOs: 26 to 28.

[Figure 12] Figure 12 shows sequences of SEQ ID NOs: 29 and 30.

[Figure 13] Figure 13 shows sequences of SEQ ID NOs: 31 and 32.

[Figure 14] Figure 14 shows sequences of SEQ ID NOs: 33 to 35.

[Figure 15] Figure 15 shows sequences of SEQ ID NOs: 36 to 38.

[Figure 16] Figure 16 shows sequences of SEQ ID NOs: 39 and 40.

[Figure 17] Figure 17 shows sequences of SEQ ID NOs: 41 to 43.

[Figure 18] Figure 18 shows sequences of SEQ ID NOs: 44 to 46.

[Figure 19] Figure 19 shows sequences of SEQ ID NOs: 47 and 48.

[Figure 20] Figure 20 shows sequences of SEQ ID NOs: 49 and 50.

Description of Embodiments

Hereafter, the present invention is described in detail.

The present invention provides an antibody that binds specifically to CD138 and an antibody-drug conjugate (ADC) having anti-tumor activity.

<Antibody>

The present invention provides an antibody that binds specifically to CD138.

The antibody of the present invention binds to a particular epitope sequence (LPEV) (SEQ ID NO: 50) of CD138 with a high binding capability. Thus, the antibody of the present invention is useful as a reagent used for detection, diagnosis, isolation, or other applications of CD138. Because of the tumor-tissue selectivity based on the binding capability, in addition, the antibody of the present invention is useful as an antibody component of an antibody-drug conjugate (ADC) having anti-tumor activity.

The term "CD138" used herein refers to the human CD138 protein, unless otherwise specified. Examples of the human CD138 proteins include a protein consisting of the amino acid sequence represented by SEQ ID NO: 49 and a protein comprising an amino acid sequence derived from the aforementioned amino acid sequence by substitution, deletion, and/or addition of 1 or several amino acids, such as 1 to 10, preferably 1 to 5, more preferably 1 or 2, and further preferably 1 amino acid and having biological activity equivalent to that of the aforementioned protein.

Examples of the antibodies of the present invention include Antibody 3 to Antibody 6 and Antibody 10 to Antibody 12 described below.

As described above, the term "Antibody 2" used herein refers to a known chimeric antibody that has a human IgG4 constant region (hereafter may be referred to as "nBT062"). In order to reduce an immunogenicity risk, in general, humanization of a chimeric antibody or the like has been attempted. In such a case, the binding capability to an antigen would be reduced or quenched. As described in the examples below, in fact, the CD138-binding capability of Antibody 7 to Antibody 9 was reduced, compared with that of Antibody 1. In order to overcome such problem, the present inventors have made various attempts to improve the binding capability. As a result, they discovered that the CD138-binding capability of Antibody 3 to Antibody 6 would be improved, compared with that of Antibody 7 to Antibody 9 as a result of a given mutation in the amino acid sequence of an antibody variable region. The heavy chain is common between Antibody 4 and Antibody 7, between Antibody 5 and Antibody 8, and between Antibody 6 and Antibody 9, and the light chain is common therebetween except for a single amino acid in a variable region. That is, a difference in a single amino acid in a variable region improves the CD138-binding capability.

The antibody of the present invention is an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16.

It is particularly preferable that the antibody of the present invention be (1) an antibody above comprising a heavy chain comprising a variable region consisting of the amino acid sequence represented by SEQ ID NO: 14 and a light chain comprising a variable region consisting of the amino acid sequence represented by SEQ ID NO: 16.

The antibody of the present invention encompasses not only the antibody comprising the heavy chain and the light chain consisting of the amino acid sequences represented by the sequence identification numbers indicated above but also an antibody comprising a variable region of a heavy chain and a variable region of a light chain consisting of the amino acid sequences represented by the sequence identification numbers indicated above and having certain mutations in an amino acid sequence (constant region) other than a variable region of at least one of the heavy chain and the light chain, provided that such antibody has the capability as the antibody of the present invention.

The heavy chain variable region encompasses not only a heavy chain variable region consisting of the amino acid sequence represented by the sequence identification number indicated above but also a heavy chain variable region consisting of a protein having certain mutations in a region other than CDR in the amino acid sequence and has activity of an antibody heavy chain variable region; i.e., CD138-binging activity. The light chain variable region encompasses not only a light chain comprising a variable region consisting of the amino acid sequence represented by the sequence identification number indicated above but also a light chain variable region consisting of a protein having certain mutations in a region other than CDR in the amino acid has activity of an antibody light chain variable region; i.e., CD138-binging activity.

The term "certain mutations" used herein refers to mutations that cause deletion, substitution, or addition of 1 or several, such as 1 to 10, preferably 1 to 5, more preferably 1 or 2, and further preferably 1 amino acid in a region other than a variable region of at least one of the heavy chain or the light chain of the amino acid sequence.

An example of a capability of the antibody of the present invention is a capability of specifically binding to CD138. Such capability is preferably a capability of specifically binding to human CD138, and it is more preferably a capability of specifically binding to an epitope sequence of human CD138 (LPEV).

Whether or not an antibody has the CD138-binding capability can be determined by a known technique.

An example of an amino acid sequence derived from the amino acid sequence represented by the sequence identification number indicated above by deletion, substitution, or addition of 1 or several amino acids is an amino acid sequence exhibiting at least 85%, preferably at least 90%, more preferably at least 91%, more preferably at least 92%, more preferably at least 93%, more preferably at least 94%, more preferably at least 95%, more preferably at least 96%, more preferably at least 97%, more preferably at least 98%, and particularly preferably at least 99% sequence identity to the amino acid sequence of a region other than the variable region of the amino acid sequence represented by the sequence identification number indicated above, which is calculated with the use of, for example, BLAST (Basic Local Alignment Search Tool at the National Center for Biological Information (e.g., default; i.e., initial parameters).

A protein comprising an amino acid sequence derived from the amino acid sequence represented by the sequence identification number indicated above by deletion, substitution, or addition of 1 or several amino acids is substantially identical to the protein consisting of the amino acid sequence represented by the sequence identification number indicated above.

The preferable antibody of the present invention is an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34

and having the capability of binding specifically to CD138;

(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and

(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138.

The particularly preferable antibody of the present invention is (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138.

Further, the preferable antibody of the present invention is an antibody selected from the group consisting of (1) to (6) below that binds specifically to CD138 and has a human IgG1 constant region:

(1) an antibody comprising a heavy chain consisting of the amino acid sequence represented by SEQ ID NO: 32 and a light chain consisting of the amino acid sequence represented by SEQ ID NO: 34;
(2) an antibody comprising a heavy chain consisting of the amino acid sequence represented by SEQ ID NO: 36 and a light chain consisting of the amino acid sequence represented by SEQ ID NO: 34;
(3) an antibody comprising a heavy chain consisting of the amino acid sequence represented by SEQ ID NO: 40 and a light chain consisting of the amino acid sequence represented by SEQ ID NO: 34;
(4) an antibody comprising a heavy chain consisting of the amino acid sequence represented by SEQ ID NO: 44 and a light chain consisting of the amino acid sequence represented by SEQ ID NO: 34;
(5) an antibody comprising a heavy chain consisting of the amino acid sequence represented by SEQ ID NO: 46 and a light chain consisting of the amino acid sequence represented by SEQ ID NO: 34; and
(6) an antibody comprising a heavy chain consisting of the amino acid sequence represented by SEQ ID NO: 48 and a light chain consisting of the amino acid sequence represented by SEQ ID NO: 34.

The more preferable antibody of the present invention is an antibody selected from the group consisting of (4) to (6) below that binds specifically to CD138 and has a human IgG1 constant region:

(4) an antibody comprising a heavy chain consisting of the amino acid sequence represented by SEQ ID NO: 44 and a light chain consisting of the amino acid sequence represented by SEQ ID NO: 34;
(5) an antibody comprising a heavy chain consisting of the amino acid sequence represented by SEQ ID NO: 46 and a light chain consisting of the amino acid sequence represented by SEQ ID NO: 34; and
(6) an antibody comprising a heavy chain consisting of the amino acid sequence represented by SEQ ID NO: 48 and a light chain consisting of the amino acid sequence represented by SEQ ID NO: 34.

The further preferable antibody of the present invention is (1) an antibody or (4) an antibody below that binds specifically to CD138 and has a human IgG1 constant region:

(1) an antibody comprising a heavy chain consisting of the amino acid sequence represented by SEQ ID NO: 32 and a light chain consisting of the amino acid sequence represented by SEQ ID NO: 34; or

(4) an antibody comprising a heavy chain consisting of the amino acid sequence represented by SEQ ID NO: 44 and a light chain consisting of the amino acid sequence represented by SEQ ID NO: 34.
The particularly preferable antibody of the present invention is (4) an antibody below that binds specifically to CD138 and has a human IgG1 constant region:
(4) an antibody comprising a heavy chain consisting of the amino acid sequence represented by SEQ ID NO: 44 and a light chain consisting of the amino acid sequence represented by SEQ ID NO: 34.

The sequence information of Antibody 1 to Antibody 12 is as shown in Table 1.

[Table 1]

| | | CDR1 | CDR2 | CDR3 | Variable region | Full-length |
|---|---|---|---|---|---|---|
| Antibody 1 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 7; amino acid sequence: SEQ ID NO: 8 | Nucleotide sequence: SEQ ID NO: 25; amino acid sequence: SEQ ID NO: 26 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 9; amino acid sequence: SEQ ID NO: 10 | Nucleotide sequence: SEQ ID NO: 27; amino acid sequence: SEQ ID NO: 28 |
| Antibody 2 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 11; amino acid sequence: SEQ ID NO: 12 | Nucleotide sequence: SEQ ID NO: 29; amino acid sequence: SEQ ID NO: 30 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 9; amino acid sequence: SEQ ID NO: 10 | Nucleotide sequence: SEQ ID NO: 27; amino acid sequence: SEQ ID NO: 28 |
| Antibody 3 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 13; amino acid sequence: SEQ ID NO: 14 | Nucleotide sequence: SEQ ID NO: 31; amino acid sequence: SEQ ID NO: 32 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |

(continued)

| | | CDR1 | CDR2 | CDR3 | Variable region | Full-length |
|---|---|---|---|---|---|---|
| Antibody 4 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 17; amino acid sequence: SEQ ID NO: 18 | Nucleotide sequence: SEQ ID NO: 35; amino acid sequence: SEQ ID NO: 36 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 5 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 19; amino acid sequence: SEQ ID NO: 20 | Nucleotide sequence: SEQ ID NO: 37; amino acid sequence: SEQ ID NO: 38 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 6 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 21; amino acid sequence: SEQ ID NO: 22 | Nucleotide sequence: SEQ ID NO: 39; amino acid sequence: SEQ ID NO: 40 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 7 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 17; amino acid sequence: SEQ ID NO: 18 | Nucleotide sequence: SEQ ID NO: 35; amino acid sequence: SEQ ID NO: 36 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 23; amino acid sequence: SEQ ID NO: 24 | Nucleotide sequence: SEQ ID NO: 41; amino acid sequence: SEQ ID NO: 42 |

(continued)

| | | CDR1 | CDR2 | CDR3 | Variable region | Full-length |
|---|---|---|---|---|---|---|
| Antibody 8 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 19; amino acid sequence: SEQ ID NO: 20 | Nucleotide sequence: SEQ ID NO: 37; amino acid sequence: SEQ ID NO: 38 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 23; amino acid sequence: SEQ ID NO: 24 | Nucleotide sequence: SEQ ID NO:41; amino acid sequence: SEQ ID NO: 42 |
| Antibody 9 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 21; amino acid sequence: SEQ ID NO: 22 | Nucleotide sequence: SEQ ID NO: 39; amino acid sequence: SEQ ID NO: 40 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 23; amino acid sequence: SEQ ID NO: 24 | Nucleotide sequence: SEQ ID NO: 41; amino acid sequence: SEQ ID NO: 42 |
| Antibody 10 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 13; amino acid sequence: SEQ ID NO: 14 | Nucleotide sequence: SEQ ID NO: 43; amino acid sequence: SEQ ID NO: 44 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 11 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 17; amino acid sequence: SEQ ID NO: 18 | Nucleotide sequence: SEQ ID NO: 45; amino acid sequence: SEQ ID NO: 46 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |

(continued)

| | | CDR1 | CDR2 | CDR3 | Variable region | Full-length |
|---|---|---|---|---|---|---|
| Antibody 12 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 21; amino acid sequence: SEQ ID NO: 22 | Nucleotide sequence: SEQ ID NO: 47; amino acid sequence: SEQ ID NO: 48 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |

The form of the antibody of the present invention is not particularly limited, provided that such antibody is a molecule that comprises a heavy chain and a light chain each comprising a variable region represented by the sequence identification number indicated above and can bind to an antigen. Examples thereof include immunoglobulin, an antigen-binding fragment or derivative of a complete antibody having the whole or some functions of the complete antibody (e.g., a Fab fragment, a F(ab')2 fragment, a single-stranded antibody fragment (scFv), a variable region (Fv), or Fab'iabody, which is a monovalent fragment of an antibody variable region obtained by treating dF(ab')2 under reducing conditions, or a bispecific antibody).

The antibody of the present invention is a humanized antibody that has a human IgG1 constant region.

The antibody of the present invention can be prepared by any of various known methods, and a method for preparing the same is not particularly limited. Examples of known methods include the hybridoma method and the phage display method.

The antibody of the present invention can be modified by a known recombination technique, provided that the effects of the present invention are not adversely affected. For example, at least 1 amino acid in an antibody constant region can be substituted with a different residue. Such modification is provided to reduce undesirable activity, such as complement-dependent cytotoxicity.

The antibody of the present invention can be prepared as a recombinant antibody. Specifically, DNA encoding each antibody region (DNA encoding a heavy chain comprising a variable region, a light chain comprising a variable region, a heavy chain constant region, or a light chain constant region) is inserted into an expression vector, a host cell is transformed using the expression vector, and the cell is cultured to prepare the antibody of the present invention. In such a case, DNA comprising DANs each encoding a relevant region ligated to each other may be operably linked to an element, such as a promoter, polyadenylation signal, or enhancer. DNA is operably linked, so that each element can function. DNA comprising a region inserted into a vector may comprise a signal peptide that accelerates antibody secretion from a host cell. After the antibody is produced, a signal peptide may be removed, so that a mature protein can be obtained. As an expression vector, a known plasmid, phage, or the like can be used. As a host cell, a prokaryotic cell, such as an *E. coli* or *Bacillus subtilis* cell, or an eukaryotic cell, such as a yeast or animal cell, can be used. In particular, use of an eukaryotic cell with a sugar chain binding to an antibody is preferable. Examples of preferable animal cells that can be used include Chinese hamster ovarian (CHO) cells and HEK293 cells. An expression vector may be introduced into a host cell by electroporation, DEAE-dextran transfection, calcium phosphate precipitation, or other means to transform the host cell. The prepared antibody may be purified by, for example, a known method involving the use of chromatography. A lysine residue at the carboxyl terminus of a heavy chain of an antibody prepared in a cultured mammalian cell is known to be deleted, and an antibody lacking a lysine residue at the carboxyl terminus of a heavy chain is within the scope of the present invention.

Any DNA may serve as DNA encoding each region of the antibody of the present invention, provided that it can express each region of the antibody of the present invention. Examples thereof include DNAs each consisting of a nucleotide represented by the sequence identification number shown in Table 1.

<Antibody-drug conjugate (ADC)>

The present invention provides an antibody-drug conjugate (ADC) represented by General formula 1 below:

Ab-(L-D)p (1)

wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents a linker;
D represents monomethyl auristatin; and
p is 1 to 12.

Ab in the ADC of the present invention is preferably the antibody described above.
L in the ADC of the present invention is a linker that connects an antibody to a drug. A configuration of a linker is not particularly limited, provided that it is cleaved by a protease and releases a drug in a cancer cell without releasing a drug in blood. An example of such linker is s cysteine-binding type dipeptide linker, and such linker is preferably 6-maleimidocaproyl-valine-citrulline-p-aminobenzyloxycarbonyl (MC-Val-Cit-PAB: MC-VC-PAB). MC-VC-PAB is known as a linker of ADC.
D in the ADC of the present invention is monomethyl auristatin (MMA). Examples of monomethyl auristatin include monomethyl auristatin E (MMAE), monomethyl auristatin D (MMAD), and monomethyl auristatin F (MMAF), with monomethyl auristatin E and monomethyl auristatin F being preferable and monomethyl auristatin E being particularly preferable. Monomethyl auristatin is a known compound, which is known to have anti-tumor activity.
MC-VC-PAB-MMAE and MC-VC-PAB-MMAF can be purchased from MedChemExpress.
The ADC of the present invention can be produced by a well-known method. In ADC, for example, a drug is bound to an antibody via a linker, and a linker is bound to an antibody via an amino or cysteine residue of an antibody. Specifically, such binding is performed by the Michael addition reaction between a sulfhydryl group and a maleimide group of cysteine, bond formation via a disulfide bond forming reaction, or amide bond formation by condensation with carboxylic acid to the lysine amino group. p in General formula 1 indicates the number of molecules constituting a drug that can bind to an antibody molecule, and the number of molecules is referred to as the drug antibody ratio (DAR). DAR can be measured by a known technique, such as hydrophobic chromatography (Methods Mol. Biol., 2013; 1045:275-83. doi: 10.1007/978-1-62703-541-5_17.).
p in the ADC of the present invention is selected from 1 to 12, preferably 1 to 8, more preferably 2 to 8, more preferably 3 to 8, more preferably 4 to 8, more preferably 5 to 8, more preferably 6 to 8, more preferably 7 to 8, and particularly preferably 8. According to another embodiment, p is preferably 2 to 8, more preferably 2 to 6, more preferably 3 to 5, and particularly preferably 4.
The antibody-drug conjugate of the present invention may be an antibody-drug conjugate comprising a uniform number of drug molecules bound to an antibody molecule or a population of antibody-drug conjugates each comprising a different number of drug molecules bound to an antibody molecule. In the case of the latter, p indicates an average number of drug molecules bound to an antibody molecule.
A preferable ADC of the present invention is an ADC represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence

represented by SEQ ID NO: 22 and a light chain comprising a variable region consisting of the amino acid sequence represented by SEQ ID NO: 16,

wherein

L represents a linker;
D represents monomethyl auristatin; and
p is 1 to 12.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin; and
p is 1 to 12.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin; and
p is 1 to 8.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence

represented by SEQ ID NO: 16; and

(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin E or monomethyl auristatin F; and
p is 1 to 8.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;

(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and

(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin E; and
p is 1 to 8.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin E; and
p is 1 to 8.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin E; and
P is 3 to 8.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein

L represents 6-maleimidocaproyl-valine-citrulline-p-aminobenzyloxycarbonyl (MC-Val-Cit-PAB: MC-VC-PAB);
D represents monomethyl auristatin E; and
p is 3 to 5.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein

L represents 6-maleimidocaproyl-valine-citrulline-p-aminobenzyloxycarbonyl (MC-Val-Cit-PAB);
D represents monomethyl auristatin E; and
p is 1 to 8.

Another preferable ADC is an ADC, which is represented by Formula 1, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein

L represents 6-maleimidocaproyl-valine-citrulline-p-aminobenzyloxycarbonyl (MC-Val-Cit-PAB);
D represents monomethyl auristatin E; and
p is 1 to 8.

Another preferable ADC is an ADC, which is represented by Formula 1, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein

L represents 6-maleimidocaproyl-valine-citrulline-p-aminobenzyloxycarbonyl (MC-Val-Cit-PAB);
D represents monomethyl auristatin E; and
p is 3 to 8.

Another preferable ADC is an ADC, which is represented by Formula 1, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein

L represents 6-maleimidocaproyl-valine-citrulline-p-aminobenzyloxycarbonyl (MC-Val-Cit-PAB);
D represents monomethyl auristatin E; and
p is 3 to 5.

Another preferable ADC is an ADC, which is represented by Formula 1, wherein Ab represents an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34,

wherein

L represents 6-maleimidocaproyl-valine-citrulline-p-aminobenzyloxycarbonyl (MC-Val-Cit-PAB);
D represents monomethyl auristatin E; and
p is 1 to 8.

Another preferable ADC is an ADC, which is represented by Formula 1, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein

L represents 6-maleimidocaproyl-valine-citrulline-p-aminobenzyloxycarbonyl (MC-Val-Cit-PAB);
D represents monomethyl auristatin E; and
p is 1 to 8.

Another preferable ADC is an ADC, which is represented by Formula 1, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein

L represents 6-maleimidocaproyl-valine-citrulline-p-aminobenzyloxycarbonyl (MC-Val-Cit-PAB);
D represents monomethyl auristatin E; and
p is 3 to 8.

Another preferable ADC is an ADC, which is represented by Formula 1, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein

L represents 6-maleimidocaproyl-valine-citrulline-p-aminobenzyloxycarbonyl (MC-Val-Cit-PAB);
D represents monomethyl auristatin E; and
p is 3 to 5.

A preferable ADC of the present invention is an ADC represented by the following formula:

[Chemical formula 1]

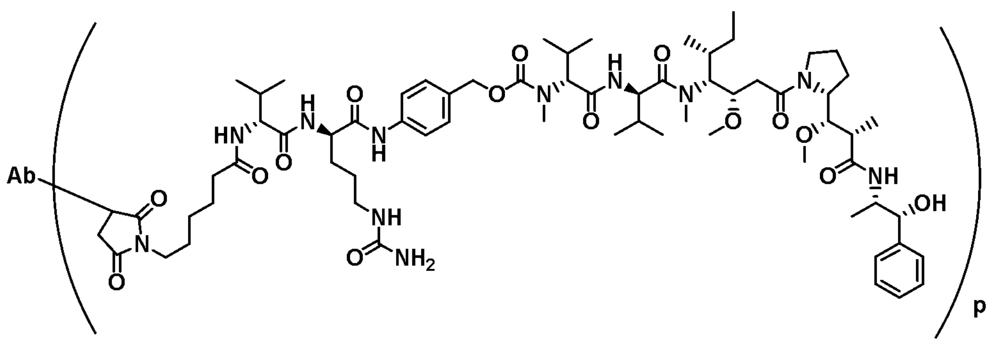

wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence

represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein
p is 3 to 8.

A more preferable ADC is represented by the formula above, wherein Ab represents (1) an antibody below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein
p is 3 to 5.

A more preferable ADC is represented by the formula above, wherein Ab represents an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 3 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 3 to 5.

A more preferable ADC is represented by the formula above, wherein Ab represents an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein
p is 3 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein
p is 3 to 5.

A preferable ADC of the present invention is an ADC represented by the following formula:

[Chemical formula 2]

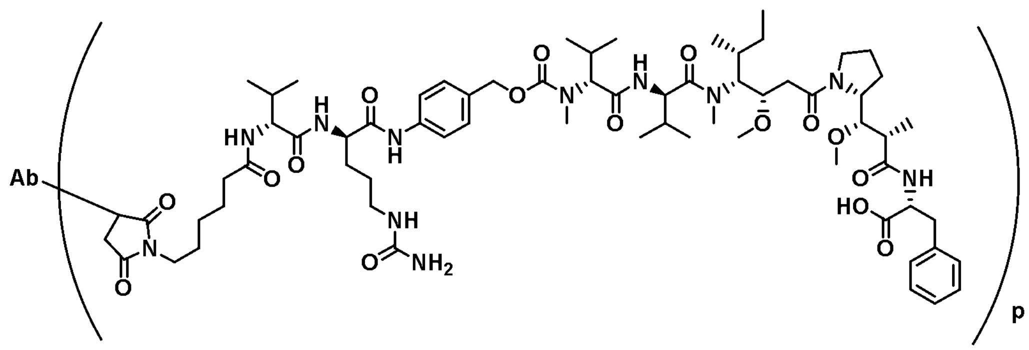

wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein
p is 3 to 8.

A more preferable ADC is represented by the formula above, wherein Ab represents (1) an antibody below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16,

wherein
p is 3 to 5.

A more preferable ADC is represented by the formula above, wherein Ab represents an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 3 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138,

wherein
p is 3 to 5.

A more preferable ADC is represented by the formula above, wherein Ab represents an antibody selected from the group consisting of (1) to (3) below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein
p is 1 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a light chain consisting of the amino acid sequence represented by SEQ ID NO: 34,

wherein
p is 3 to 8.

A more preferable ADC is represented by the formula above, wherein Ab represents (1) an antibody below that binds specifically to CD138 and has a human IgG1 constant region:

(1) an antibody comprising a heavy chain consisting of the amino acid sequence represented by SEQ ID NO: 44 and a

light chain consisting of the amino acid sequence represented by SEQ ID NO: 34,

wherein
p is 3 to 5.

Hereafter, a linker-drug structure and a linker structure used in a preferable ADC of the present invention are shown. In a figure of a linker, a linker binds to an antibody on its lefthand side and it binds to a drug on its right-hand side at the sites indicated by broken lines.

ADC3, ADC8 to ADC14, ADC16, and ADC17 (MC-Val-Cit-PAB-MMAE)

[Chemical formula 3]

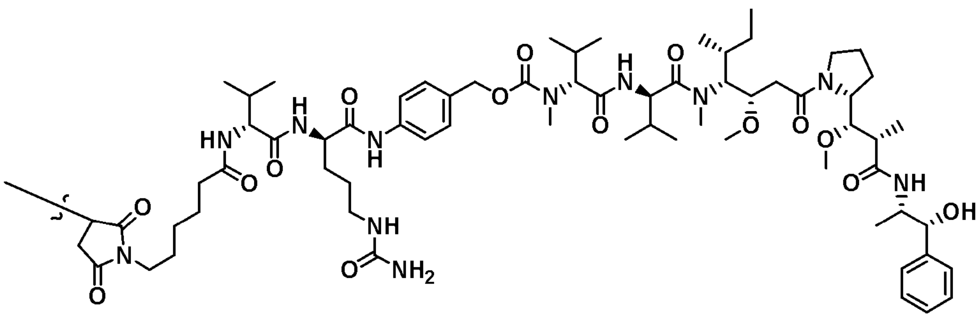

MC-Val-Cit-PAB

[Chemical formula 4]

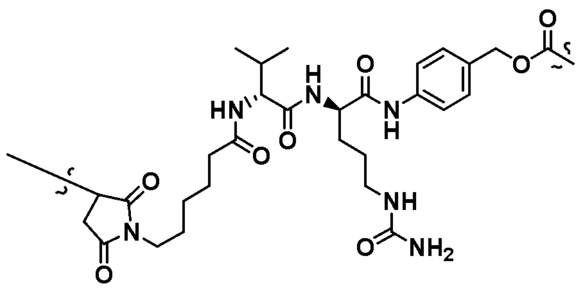

MMAE

[Chemical formula 5]

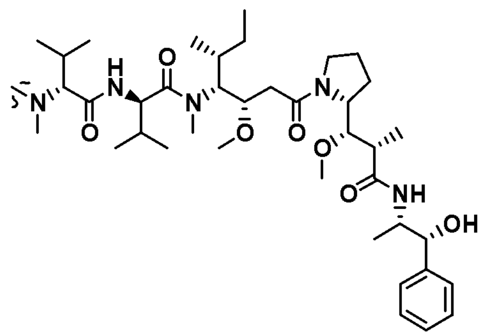

ADC4 (MC-Val-Cit-PAB-MMAF)

[Chemical formula 6]

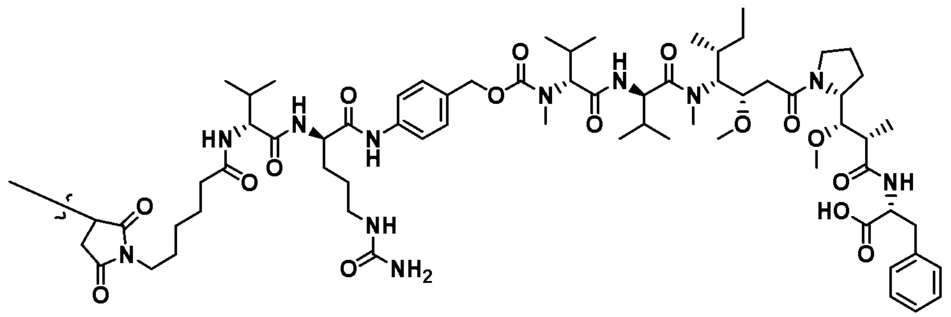

MC-Val-Cit-PAB

[Chemical formula 7]

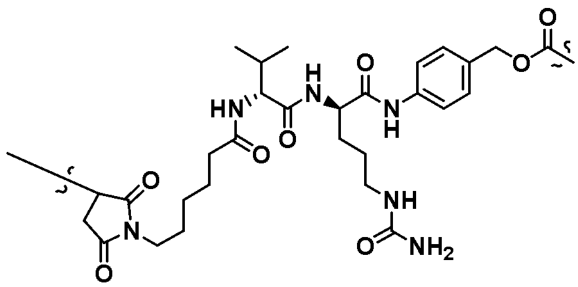

MMAF

[Chemical formula 8]

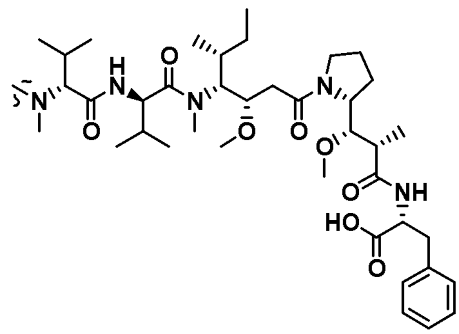

The ADC and the antibody of the present invention have stable physical properties as described in the examples below. Physical properties can be evaluated with various indicators in terms of, for example, the susceptibility to denaturation or aggregation. For example, evaluation can be made by determining the denaturation midpoint temperature $T_m2$, the aggregation initiation temperature $T_{agg}266$, or the particle size distribution using UNcle (UNCHAINED LABS). The ADC of the present invention preferably has stable physical properties, more preferably the denaturation midpoint temperature $T_m2$ of 80°C or higher or the aggregation initiation temperature $T_{agg}266$ of 70°C or higher, and particularly preferably the denaturation midpoint temperature $T_m2$ of 80°C or higher or the aggregation initiation temperature $T_{agg}266$

of 70°C or higher.

The ADC and the antibody of the present invention can be in the form of pharmaceutically acceptable salt. Examples of pharmaceutically acceptable salt include, but are not particularly limited to, inorganic acid salt, organic acid salt, alkali metal salt, alkaline earth metal salt, and organic amine salt.

When the ADC and the antibody of the present invention have an isomer, such as an optical isomer, a stereoisomer, a rotational isomer, or a tautomer, the ADC and the antibody include a mixture of any isomers, unless otherwise specified.

<Anti-tumor agent>

The present invention also includes an anti-tumor agent comprising, as an active ingredient, the ADC.

Types of cancers on which the antibody-drug conjugate (ADC) of the present invention exerts anti-tumor effects are not particularly limited, provided that the cancer expresses CD138. Specific examples include multiple myeloma, acute myelocytic leukemia, gastric cancer, pancreatic cancer, large bowel cancer, hepatic cancer, lung cancer, breast cancer, skin cancer, head and neck cancer, oral cavity cancer, laryngeal cancer, esophageal cancer, gallbladder cancer, cervix cancer, endometrial cancer, ovarian cancer, mesothelioma, renal cell cancer, urothelial cancer, prostate cancer, skin cancer, bone and soft tissue tumor, rhabdomyosarcoma, brain tumor, malignant neurilemmoma, neuroendocrine tumor, and thyroid gland cancer. Cancer is preferably solid cancer, more preferably gastric cancer, pancreatic cancer, large bowel cancer, hepatic cancer, lung cancer, breast cancer, skin cancer, oral cavity cancer, laryngeal cancer, esophageal cancer, or urothelial cancer, and particularly preferably gastric cancer, pancreatic cancer, large bowel cancer, lung cancer, breast cancer, or urothelial cancer.

The ADC of the present invention is incorporated into and internalized in the cancer cell and it exerts therapeutic effects thereon.

The route of administration of the anti-tumor agent comprising the ADC of the present invention is not limited, and the anti-tumor agent can be administered through the oral, parenteral, or other route. Examples of parenteral administration include intravenous administration, intramuscular administration, and subcutaneous administration. Alternatively, the anti-tumor agent may be administered through the transmucosal (e.g., sublingual or buccal), percutaneous, or rectal route. The anti-tumor agent may be administered systemically or topically on the cancer tissue. Examples of dosage forms of formulations include tablets, capsules, pills, powders, granules, and injection formulations. Oral formulations, such as tablets, capsules, pills, powders, or granules, can be prepared with the use of additives, and examples of additives include: excipients, such as glucose, sucrose, lactose, and mannitol; disintegrators, such as starch and sodium alginate; lubricants, such as magnesium stearate and talc; binders, such as polyvinyl alcohol, gelatin, and hydroxypropyl cellulose; surfactants, such as fatty acid ester; and plasticizers, such as glycerin. Injection formulations can be prepared with the use of additives, and examples of additives include: water; saccharides, such as sucrose, sorbitol, xylose, trehalose, and fructose; sugar alcohol, such as sorbitol, mannitol, and xylitol; buffers, such as phosphate buffer, citrate buffer, and glutamate buffer; and surfactants, such as fatty acid ester.

A dose of the ADC to a patient who is in need of treatment varies depending on age, gender, severity, and other conditions of the patient to which the ADC is to be administered, and the physician can determine an adequate dose. For example, the ADC may be administered at 0.05 to 10 mg/kg/body weight, and preferably 0.1 to 2 mg/kg/body weight, per instance. Also, administration may be performed at 0.001 nM to 1000 nM, and preferably 0.001 nM to 100 nM, per instance, at, for example, the MMAE equivalent dose.

While administration may be performed once daily, administration may be performed 2, 3, 4, or more separate instances daily. In such a case, administration may be performed at adequate intervals for 1 day to several years.

The anti-tumor agent comprising, as an active ingredient, the ADC of the present invention can be used in combination with other anti-tumor agents or other therapeutic techniques. When the anti-tumor agent is "used in combination" in the present invention, 2 or more active ingredients are used in combination, and such use can be determined based on the product document of the pharmaceutical preparation, administration regimen, and the like. Accordingly, use in combination can be regarded as the use of an active ingredient that is administered as a part of an administration regimen. When 2 or more active ingredients are administered to a patient, accordingly, separate administration forms may be adopted for each active ingredient, or some or all active ingredients may be formulated in a single administration form. When separate administration forms are adopted for each active ingredient, the active ingredients can be administered simultaneously or successively at any order, provided that useful effects can be achieved by the 2 or more agents. The intervals of administration in the case of successive administration can be adequately determined, provided that useful effects can be achieved by the use of 2 or more agents in combination.

Examples of other therapeutic techniques include surgery and radiotherapy.

Anti-tumor agents other than the ADC of the present invention are not particularly limited, provided that such agents are used for treatment of cancer. Such agents may be low-molecular-weight agents or biopharmaceuticals, such as antibodies. Specific examples include chemotherapeutic agents, such as alkylating agents, antimetabolites, microtubule-acting agents, platinum-containing drugs, topoisomerase inhibitors, platinating agents, and anti-tumor antibiotics, molecular-targeted drugs, such as tyrosine kinase inhibitors, mTOR inhibitors, proteasome inhibitors, HDAC inhibitors, CKD inhibitors, and PARP inhibitors, hormones, and immune checkpoint inhibitors.

[Examples]

Hereafter, the present invention is described in detail with reference to the examples, although the present invention is not limited thereto.

[Example 1: Preparation of CD138 monoclonal antibody (mAB) by recombinant DNA method

cDNA constructs were prepared from the genes of the heavy chains and the light chains encoding Antibody 1 to 12 shown in Table 2. cDNA constructs encoding the heavy chains and the light chains were integrated into the pcDNA3 plasmid (Life Technologies) and introduced into HEK293 cells in combination with clones to forcibly express the heavy chains and the light chains. After the culture supernatant was filtered through a 0.22-μm filter, Protein A purification and gel filtration purification were performed to obtain the anti-CD138 antibody.

[Table 2]

| | | CDR1 | CDR2 | CDR3 | Variable region | Full-length |
|---|---|---|---|---|---|---|
| Antibody 1 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 7; amino acid sequence: SEQ ID NO: 8 | Nucleotide sequence: SEQ ID NO: 25; amino acid sequence: SEQ ID NO: 26 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 9; amino acid sequence: SEQ ID NO: 10 | Nucleotide sequence: SEQ ID NO: 27; amino acid sequence: SEQ ID NO: 28 |
| Antibody 2 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 11; amino acid sequence: SEQ ID NO: 12 | Nucleotide sequence: SEQ ID NO: 29; amino acid sequence: SEQ ID NO: 30 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 9; amino acid sequence: SEQ ID NO: 10 | Nucleotide sequence: SEQ ID NO: 27; amino acid sequence: SEQ ID NO: 28 |
| Antibody 3 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 13; amino acid sequence: SEQ ID NO: 14 | Nucleotide sequence: SEQ ID NO: 31; amino acid sequence: SEQ ID NO: 32 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |

(continued)

| | | CDR1 | CDR2 | CDR3 | Variable region | Full-length |
|---|---|---|---|---|---|---|
| Antibody 4 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 17; amino acid sequence: SEQ ID NO: 18 | Nucleotide sequence: SEQ ID NO: 35; amino acid sequence: SEQ ID NO: 36 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 5 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 19; amino acid sequence: SEQ ID NO: 20 | Nucleotide sequence: SEQ ID NO: 37; amino acid sequence: SEQ ID NO: 38 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 6 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 21; amino acid sequence: SEQ ID NO: 22 | Nucleotide sequence: SEQ ID NO: 39; amino acid sequence: SEQ ID NO: 40 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 7 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 17; amino acid sequence: SEQ ID NO: 18 | Nucleotide sequence: SEQ ID NO: 35; amino acid sequence: SEQ ID NO: 36 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 23; amino acid sequence: SEQ ID NO: 24 | Nucleotide sequence: SEQ ID NO: 41; amino acid sequence: SEQ ID NO: 42 |

(continued)

| | | CDR1 | CDR2 | CDR3 | Variable region | Full-length |
|---|---|---|---|---|---|---|
| Antibody 8 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 19; amino acid sequence: SEQ ID NO: 20 | Nucleotide sequence: SEQ ID NO: 37; amino acid sequence: SEQ ID NO: 38 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 23; amino acid sequence: SEQ ID NO: 24 | Nucleotide sequence: SEQ ID NO:41; amino acid sequence: SEQ ID NO: 42 |
| Antibody 9 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 21; amino acid sequence: SEQ ID NO: 22 | Nucleotide sequence: SEQ ID NO: 39; amino acid sequence: SEQ ID NO: 40 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 23; amino acid sequence: SEQ ID NO: 24 | Nucleotide sequence: SEQ ID NO: 41; amino acid sequence: SEQ ID NO: 42 |
| Antibody 10 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 13; amino acid sequence: SEQ ID NO: 14 | Nucleotide sequence: SEQ ID NO: 43; amino acid sequence: SEQ ID NO: 44 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |
| Antibody 11 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 17; amino acid sequence: SEQ ID NO: 18 | Nucleotide sequence: SEQ ID NO: 45; amino acid sequence: SEQ ID NO: 46 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |

(continued)

| | | CDR1 | CDR2 | CDR3 | Variable region | Full-length |
|---|---|---|---|---|---|---|
| Antibody 12 | IgH | NYWIE (SEQ ID NO: 1) | EILPGTGR TIYNEKFKG (SEQ ID NO: 2) | RDYYGNFYYAMDY (SEQ ID NO: 3) | Nucleotide sequence: SEQ ID NO: 21; amino acid sequence: SEQ ID NO: 22 | Nucleotide sequence: SEQ ID NO: 47; amino acid sequence: SEQ ID NO: 48 |
| | IgK | SASQGINNYLN (SEQ ID NO: 4) | YTSTLQS (SEQ ID NO: 5) | QQYSKLPRT (SEQ ID NO: 6) | Nucleotide sequence: SEQ ID NO: 15; amino acid sequence: SEQ ID NO: 16 | Nucleotide sequence: SEQ ID NO: 33; amino acid sequence: SEQ ID NO: 34 |

[Example 2: Binding of anti-CD138 antibody to cancer cell]

Cells of the human pancreatic cancer cell line AsPC-1 (Sumitomo Dainippon Pharma Co., Ltd.) were washed with PBS and then collected using a cell dissociation solution (Sigma). The number of the cells was counted and collected at $5 \times 10^5$ cells/well on a 96-well plate. The anti-CD138 antibody obtained in Example 1 and the human IgG isotype control (Invitrogen) were diluted to the final concentration of 0.001 to 100 μg/ml using the autoMACS Running Buffer-MACS Separation Buffer (Miltenyi Biotec), added to the AsPC-1 cells, and then allowed to stand at 4°C for 30 minutes. After the reaction with the anti-CD138 antibody obtained in Example 1 and with the human IgG isotype control, the cells were washed with PBS (pH 7.4), and the antibody goat-anti-human IgG Fc-APC (BioLegend) diluted in the autoMACS Running Buffer-MACS Separation Buffer was then added thereto. After the reaction with the secondary antibody, the cells were washed with PBS (pH 7.4), and the APC signal (mean fluorescence intensity (MFI)) was detected using a flow cytometer (CytoFLEX S, Beckman Coulter). As a result, the binding capability of the evaluated Antibody 1 and Antibody 3 to Antibody 9 to the AsPC-1 cells was observed (Figure 1). The binding capability of Antibody 7 to Antibody 9 to the AsPC-1 cells was found to be inferior to that of Antibody 1.

[Example 3: Prediction of immunogenicity risk of antibody]

Based on the amino acid sequence of a variable region of Antibody 1 and the amino acid sequence of a variable region of Antibody 10, the immunogenicity risk of the antibodies was predicted by the Epibase *in silico* profiling (Lonza). As a result, the immunogenicity risk of Antibody 10 was found to be lower than that of Antibody 1.

[Example 4: Preparation of antibody-drug conjugate with anti-CD138 antibody]

The ADC of the present invention can be prepared by allowing a drug linker to be added to ADC to react with an antibody having an amino group and a sulfhydryl group in the manner described below. Concerning the ADCs obtained with the use of the anti-CD138 antibody, Figures 2-1, 2-2, and 2-3 show combinations of antibodies and drug linkers bound thereto. The prepared ADCs are subjected to concentration, buffer exchange, purification, and measurement of the antibody concentration and the average number of drugs bound to an antibody molecule in accordance with the common operations described below to identify the ADCs.

Common operation A: Concentration of antibody or ADC solution

The antibody or ADC solution was introduced into the Amicon Ultra container (100,000 MWCO, Millipore Corporation) and concentrated by centrifugation (at 2000 G to 15000 G for 5 to 20 minutes) using a centrifuge (himac CF5RE, HITACHI).

Common operation B: Measurement of antibody concentration

Antibody concentration was measured with the use of the UV photometer (Nanodrop 8000, Thermo Fisher Scientific Inc.) in accordance with the manufacturer's instructions.

Common operation C: Antibody/ADC buffer exchange and purification

The Zeba(™) Spin Desalting Column (7 K MWCO to 40 K MWCO, Cat. 89890, Thermo Scientific) was equilibrated with D-PBS (Nacalai Tesque Inc.) in accordance with the manufacturer's instructions. After the antibody/ADC solution was mounted, the antibody/ADC was eluted with D-PBS in the amount prescribed by the manufacturer, and the concentration was measured in accordance with the common operation B.

Common operation D: Measurement of average number of drugs bound to an antibody molecule in ADC

The drug-antibody ratio was analyzed by native size-exclusion chromatography mass spectrometry (SEC-MS) or hydrophobic interaction chromatography with UV detection (HIV-UV).

The SEC-MS analysis enables measurement of the drug-antibody ratio based on a mass change resulting from binding of a drug linker to an antibody. ADC was injected at 2 mg/ml into Acquity LC Xevo G2-S Q Tof MS (Waters Corporation), and ADC was eluted from the column by isocratic elution (100 mM aqueous ammonium acetate solution or 100 mM aqueous ammonium acetate solution/isopropanol (9/1), 20 min). The obtained data were analyzed using MassLynx software (Waters Corporation). Measurement was performed using the ACQUITY UPLC Protein BEH SEC Column, 200 Å, 1.7 μm, 4.6 mm × 150 mm (Waters) at a flow rate of 0.1 to 0.3 ml/min and column temperature of 25°C.

HIC-UV analysis was performed in the manner described below. The sample was diluted to 1 mg/ml with D-PBS(-). A 10-μl fraction of the solution was tested by liquid chromatography under the conditions described below. The peak area of each sample solution was measured by the automatic integration method, and the amount thereof was determined by the area percentage method.

Test conditions

Detector: Ultraviolet photometer (measurement wavelength: 220 nm)
Column: AdvanceBio HIC 4.6 × 100 mm, 3.5 μm (Agilent Technologies)
Column temperature: constant near 30°C
Mobile phase A: 50 mmol/l sodium phosphate buffer (pH 7.0)
Mobile phase B: 2 mM ammonium sulfate in 50 mmol/l sodium phosphate buffer (pH 7.0)
Mobile phase C: 2-propanol
Mobile phase liquid delivery: the mixing ratio of Mobile phase A, Mobile phase B, and Mobile phase C was changed as shown in Table 3 and concentration-gradient was controlled

[Table 3]

| Time after injection (min) | Mobile phase A (vol%) | Mobile phase B (vol%) | Mobile phase C (vol%) |
|---|---|---|---|
| 0 to 15 | 50→75 | 45→0 | 5→25 |
| 15 to 20 | 75 | 0 | 25 |
| 20 to 21 | 75→50 | 0→45 | 25→5 |
| 21 to 31 | 50 | 45 | 5 |

Flow rate: 0.5 ml/min
Range of area measurement: up to 31 minutes after injection after the solvent peak
Reagent/test solution
- 50 mmol/l sodium phosphate buffer (pH 7.0)

Phosphoric acid (3.425 ml) was added to 980 ml of water, and a pH of the resultant was adjusted to 7.0 with the use of 5 mol/l NaOH.

- 2 mM ammonium sulfate in 50 mmol/l sodium phosphate buffer (pH 7.0)

To 132.14 g of ammonium sulfate, 500 ml of 50 mmol/l sodium phosphate buffer (pH 7.0) was added and dissolved therein.

- 0.02% (W/V) Tween 20

Water (100 ml) was added to 0.2 g of Tween 20 and dissolved therein. A 10-ml fraction of the solution was mixed with 90 ml of water.

[Production Example 1: Antibody-drug conjugate 1 (ADC1)]

Step 1: Conjugation of antibody to drug linker

To a solution containing 2 mg of Antibody 1 prepared in Example 1, 9.6 molar equivalents of a DMSO solution containing 10 mM SMCC-DM1 (MedChemExpress) was added per one molecule of the antibody at room temperature, and N,N-dimethylacetamide (DMA) was added thereto to a final concentration of 10%. The resultant was agitated using a rotator at room temperature for 24 hours, and a drug linker was allowed to bind to an antibody. Thus, an antibody-drug conjugate 1 (ADC1) was obtained.

Step 2: Purification and physical property evaluation

A solution containing the antibody-drug conjugate 1 (ADC1) obtained in Step 1 was subjected to purification and measurement of the concentration in accordance with the common operation C, and the property values indicated below were obtained in accordance with the common operation D. The product was tested by dynamic light scattering to confirm that an aggregate would not be detected.
Antibody concentration: 1.94 mg/ml; average number of drugs bound to an antibody molecule (n): 1.5

[Production Example 2: Antibody-drug conjugate 2 (ADC2)]

Step 1: Conjugation of antibody to drug linker

To a solution comprising 65 mg of Antibody 2 prepared in Example 1, 16 molar equivalents of a DMSO solution comprising 10 mM SPDB-DM4 (MedChemExpress) was added per one molecule of the antibody at room temperature, and DMA was added thereto to a final concentration of 10%. The resultant was agitated using a rotator at room temperature for 24 hours, and a drug linker was allowed to bind to an antibody. Thus, an antibody-drug conjugate 2 (ADC2) was obtained.

Step 2: Purification and physical property evaluation

A solution containing the antibody-drug conjugate 2 (ADC2) obtained in Step 1 was subjected to purification and measurement of the concentration in accordance with the common operation C, and the property values indicated below were obtained in accordance with the common operation D-2. The product was tested by dynamic light scattering to confirm that it comprised an aggregate.
Antibody concentration: 5.66 mg/ml; average number of drugs bound to an antibody molecule (n): 2.7

[Production Examples 3 to 14: Antibody-drug conjugates 3 to 14 (ADC3 to ADC14)]

Step 1: Antibody reduction

To solutions each comprising Antibodies 1, 3 to 6, and 10 to 12 prepared in Example 1, an aqueous solution of 5 mM TCEP (20 molar equivalents per one molecule of the antibody) was added, and the resultants were incubated at 37°C for 2 hours to reduce disulfide bonds in the hinge regions of the antibodies.

Step 2: Conjugation of antibody to drug linker

To the solution obtained in Step 1, 9.6 molar equivalents of DMSO solutions each containing 10 mM MC-VC-PAB-MMAE (MedChemExpress), MC-VC-PAB-MMAF (MedChemExpress), MC-GGFG-Dxd (MedChemExpress), MC-GGFG-Dx8951 (MedChemExpress), or MC-VA-PBD (MedChemExpress) were added per one molecule of the antibody at room temperature. The resultants were agitated using a rotator for 24 hours, and a drug linker was allowed to bind to an antibody. Thus, the antibody-drug conjugates 3 to 14 (ADC3 to ADC14) shown in Figures 2-1, 2-2, and 2-3 were obtained.

Step 3: Purification and physical property evaluation

The solutions each comprising any of the antibody-drug conjugates 3 to 14 (ADC3 to ADC14) obtained in Step 2 were subjected to purification, concentration, and measurement of the concentration in accordance with the common operations A and C, and the property values indicated in Table 4 were obtained in accordance with the common operation D. The products were tested by dynamic light scattering to confirm the presence or absence of aggregates, and the results shown in Table 4 were obtained.

[Table 4]

| Production Example | Antibody | Linker-Payload | DAR | Aggregate |
|---|---|---|---|---|
| 1 | Antibody 1 | SMCC-DM1 | 1.5 | Not formed |
| 2 | Antibody 2 | SPDB-DM4 | 2.7 | Formed |
| 3 | Antibody 1 | MC-VC-PAB-MMAE | 7.1 | Not formed |
| 4 | Antibody 1 | MC-VC-PAB-MMAF | 2.2 | Not formed |
| 5 | Antibody 1 | MC-GGFG-Dxd | 4.4 | Not formed |
| 6 | Antibody 1 | MC-GGFG-Dx8951 | 6.6 | Not formed |
| 7 | Antibody 1 | MC-VA-PBD | 2.5 | Formed |
| 8 | Antibody 3 | MC-VC-PAB-MMAE | 7.9 | Not formed |
| 9 | Antibody 4 | MC-VC-PAB-MMAE | 7.8 | Not formed |
| 10 | Antibody 5 | MC-VC-PAB-MMAE | 7.8 | Not formed |
| 11 | Antibody 6 | MC-VC-PAB-MMAE | 7.8 | Not formed |
| 12 | Antibody 10 | MC-VC-PAB-MMAE | 7.9 | Not formed |
| 13 | Antibody 11 | MC-VC-PAB-MMAE | 7.9 | Not formed |
| 14 | Antibody 12 | MC-VC-PAB-MMAE | 7.9 | Not formed |

[Example 5: Evaluation of *ex vivo* stability of ADC in plasma]

Stability of ADC1 to ADC6 and ADC8 to ADC14 prepared in Example 4 in mouse and human plasma samples was evaluated.

Step 1: Incubation in plasma

ADCs adjusted to 1.0 or 0.5 mg/ml were added to the final concentration of 20 or 10 μg/ml to the human and BALB/c mouse plasma samples (anticoagulant: heparin sodium) and the resultants were incubated for 48 hours.

Step 2: Pretreatment

- In the case of an ADC comprising MMAE or MMAF as a drug, an incubation sample was deproteinated with ethanol to extract a free drug from the ADC.
- In the case of an ADC comprising Dxd or Dx8951 as a drug, an incubation sample was deproteinated with acetonitrile/methanol (9/1, v/v), an extract of the free drug was diluted to 2-fold with 1% formic acid, and the resultant was incubated for 1 hour.
- In the case of an ADC comprising SMCC-DM1 as a linker-drug, an incubation sample was reduced with TCEP and deproteinated with acetonitrile/methanol (9/1, v/v) to extract DM1 released from the ADC.
- In the case of an ADC comprising SPDB-DM4 as a linker-drug, an incubation sample was introduced into the MonoSpinProA column (GL sciences) to extract an ADC. The ADC extract was reduced with TCEP to remove DM4 that had bound to the ADC. The supernatant liquid was deproteinated with acetonitrile/methanol (9/1, v/v) to extract DM4.

Step 3: LC/MS/MS assay

The sample solutions pretreated in accordance with Step 2 were subjected to LC/MS/MS assays, and payload release (%) was determined in accordance with the equations indicated below.

ADC other than ADC comprising SPDB-DM4:

$$\text{Payload release (\%)} = \frac{\text{Free payload concentration (nmol/L) in incubation solution}}{\text{ADC concentration (}\mu\text{g/mL) in incubation solution} \times 1000 \times 1000 / \text{ADC MW} \times \text{DAR}} \times 100$$

ADC comprising SPDB-DM4:

$$\text{Payload release (\%)} = 100 - \frac{\text{Measured value of incubtion sample}}{\text{Measured value of 0 hr control sample}} \times 100$$

The results demonstrate that ADC3, ADC5, ADC6, and ADC8 to ADC14 are stable with the payload release of less than 5% in mouse and human plasma samples (Table 5).

[Table 5]

| | Free Payload Release (%) _37°C, 48h | |
|---|---|---|
| | Human | Mouse |
| ADC1 | 11.12 | 6.31 |
| ADC2 | 8.29 | 23.96 |
| ADC3 | 0.02 | 2.22 |
| ADC4 | 1.73 | 118.00 |
| ADC5 | 0.38 | 1.22 |
| ADC6 | <0.06 | <0.06 |
| ADC8 | 0.07 | 4.22 |
| ADC9 | 0.07 | 4.82 |
| ADC10 | 0.06 | 4.46 |
| ADC11 | 0.05 | 4.41 |
| ADC12 | 0.03 | 1.79 |
| ADC13 | 0.04 | 1.69 |
| ADC14 | 0.04 | 1.79 |

[Example 6: Cytotoxicity mediated by ADC]

Cells of the human pancreatic cancer cell line Capan-1 (HSRRB) were washed with PBS and then collected using a cell dissociation solution (Sigma). The number of the cells of the human T cell leukemia Jurkat cell line (JCRB) was counted and collected at $1 \times 10^6$ cells/well on a 96-well plate. Antibody 1 obtained in Example 1 and the human IgG isotype control (Invitrogen) were diluted to the final concentration of 15 $\mu$g/ml using the autoMACS Running Buffer-MACS Separation Buffer (Miltenyi Biotec), added to Capan-1 and Jurkat, and then allowed to stand at room temperature for 30 minutes. After the reaction with the antibody, the cells were washed with PBS (pH 7.4), and the antibody goat-anti-human IgG Fc-APC (BioLegend) diluted in the autoMACS Running Buffer-MACS Separation Buffer was then added thereto. After the reaction with the secondary antibody, the cells were washed with PBS (pH 7.4), and the APC signal (mean fluorescence intensity (MFI)) was detected using a flow cytometer (CytoFLEX S, Beckman Coulter). The results demonstrate that Antibody 1 would bind to the Capan-1 cells but would not bind to the Jurkat cells (Figure 3).

ADC1 to ADC14 were prepared in accordance with the procedure described in Example 4, and the cytotoxicity thereof against the pancreatic cancer cell line Capan-1 and the human T cell leukemia Jurkat was evaluated by the CellTiter-Glo 2.0 luminescent cell viability assay (Promega). The cells were seeded at 1,000 cells/well on a 96-well plate, the ADCs were added, and culture was then performed at 37°C for 5 days. Thereafter, the reagent for CellTiter-Glo 2.0 luminescent cell viability assay was added. The relative luminescence was measured using the EnSpire Multimode Plate Reader (PerkinElmer), the data were analyzed using the XLfit software (IDBS), and $IC_{50}$ for each cancer cell line was determined (Table 6). The results demonstrate that ADC2, ADC3, ADC4, and ADC7 to ADC14 exerted the cytotoxicity against the Capan-1 cells to which Antibody 1 had bound. In contrast, ADC2 and ADC7 exerted the cytotoxicity against the

Jurkat cells to which Antibody 1 did not bind. Such cytotoxicity is considered to be induced by the antigen-independent mechanism. The results indicate that ADC3, ADC4, and ADC8 to ADC14 have the antigen-dependent mechanism and such ADCs are useful as anti-tumor agents.

[Table 6]

| | $IC_{50}$ (ng/ml) | |
|---|---|---|
| | Capan-1 (CD138-positive) | Jurkat (CD138-negative) |
| ADC1 | 8385 | 7996 |
| ADC2 | 248 | 274 |
| ADC3 | 13 | 2357 |
| ADC4 | 165 | >10,000 |
| ADC5 | >10,000 | 2016 |
| ADC6 | >10,000 | >10,000 |
| ADC7 | 20 | 10 |
| ADC8 | 17 | 3124 |
| ADC9 | 18 | 3260 |
| ADC10 | 29 | 3674 |
| ADC11 | 31 | 3592 |
| ADC12 | 21 | 4028 |
| ADC13 | 40 | 4377 |
| ADC14 | 57 | 4761 |

[Example 7: Anti-tumor effects of ADC (*in vivo*)]

Cancer-carrying animal models were prepared using the cells of the pancreatic cancer cell line (Capan-1). At the outset, the cells were prepared and inoculated subcutaneously into the right chest of nude mice ($1 \times 10^7$ cells/100 μl). When the volume of the inoculated tumor was increased to approximately 100 to 300 $mm^3$, the nude mice were randomly divided into a control group (PBS administration) and test groups, ADC2 was injected intravenously at 1.25 mg/kg, 5 mg/kg, or 20 mg/kg, and ADC3 was injected intravenously at 20 mg/kg. Administration was performed once a week. The tumor volume was measured two times a week, and the tumor volume was calculated in accordance with the equation indicated below:

$$\text{Tumor volume (mm}^3\text{)} = (\text{long axis} \times \text{short axis}^2)/2$$

As shown in Figure 4, it was confirmed 7 days after the initiation of the administration that the tumor growth was more inhibited and the tumor volume was further reduced in the ADC3 administration group, compared with the control group. In the ADC3 administration group, body weight loss or other changes were not observed. In the ADC2 administration group, in contrast, the tumor growth was inhibited as a result of administration at 20 mg/kg, although anti-tumor effects were not observed as a result of administration at 1.25 mg/kg or 5 mg/kg. In the ADC2 administration group, in addition, body weight loss was observed in a dose-dependent manner. The results demonstrate that the ADC combination of the present invention exerts more significant tumor growth inhibitory effects compared with the antibody-linker-payload combination of BT062 (ADC2), which is a prior art technique, and that the ADC of the present invention has the excellent balance between the anti-tumor effects and the toxicity.

[Example 8: Anti-tumor effects of ADC (*in vivo*)]

Cancer-carrying animal models were prepared using the cells of the pancreatic cancer cell line (Capan-1). At the outset, the cells were prepared and inoculated subcutaneously into the right chest of nude mice ($1 \times 10^7$ cells/100 μl). When the volume of the inoculated tumor was increased to approximately 100 to 200 $mm^3$, the nude mice were randomly

divided into a control group (PBS administration) and test groups, and ADC3, ADC12, ADC13, and ADC14 were injected intravenously thereinto at 1 mg/kg. Administration was performed once a week. The tumor volume was measured two times a week, and the tumor volume was calculated in accordance with the equation indicated below:

$$\text{Tumor volume (mm}^3\text{)} = (\text{long axis} \times \text{short axis}^2)/2$$

As shown in Figure 5, it was confirmed 7 days after the initiation of the test that the tumor growth was more inhibited in all the ADC administration groups, compared with the control group. In all the ADC administration groups, in addition, body weight loss was not observed. The results demonstrate that the combinations of ADC3, ADC12, ADC13, and ADC14 of the present invention exert equivalent anti-tumor effects *in vivo.*

[Production Example 15: Antibody-drug conjugate 15 (ADC15)]

In accordance with the production method described in Patent Literature 1, an ADC was prepared with the use of Antibody 2 prepared in Example 1 as the antibody and SPDB-DM4 as the linker and the payload.

Antibody concentration: 2.71 mg/ml, the average number of drugs bound to an antibody molecule (n): 3.39 (SEC-MS analysis: DAR0 (4.42%), DAR1 (11.39%), DAR2 (15.97%), DAR3 (20.62%), DAR4 (20.20%), DAR5 (15.78%), DAR6 (8.31%), DAR7 (2.65%), DAR8 (0.67%))

[Production Example 16: Antibody-drug conjugate 16 (ADC16)]

The solution of Antibody 10 prepared in Example 1 (12.02 mg/ml, 32.0 ml) was diluted with the addition of 30.2 ml of a 50 mM phosphate buffer with a pH adjusted to 4.0. While agitating the solution at room temperature, 6.944 ml of an aqueous solution of 0.85% $H_3PO_4$ was added. A pH of the solution was 4.41. While agitating the solution at room temperature, 1.7828 ml of a solution of TCEP ▪ HCl in 5 mM D-PBS(-) was added, and the reaction solution was agitated at 37°C for 2 hours. A solution of MC-VC-PAB-MMAE in 10 mM DMSO (1.7828 ml) was added, the resultant was agitated at room temperature for 1 hour, 72.7 ml of D-PBS(-) (pH 7) was added to the reaction solution, and the resultant was agitated at room temperature for an additional 1 hour. A solution of TCEP ▪ HCl in 5 mM D-PBS(-) (2.0064 ml) was further added. After the reaction solution was agitated at 37°C for 1.5 hours, 0.8914 ml of a solution of MC-VC-PAB-MMAE in 10 mM DMSO was added, and the resultant was agitated at room temperature for 1 hour.

The reaction solution obtained was filtered through the Stericup-HV Sterile Vacuum Filtration System (0.45 μm, PVDF, Merck Millipore). The filtrate was subjected to ultrafiltration with the use of Pellicon XL50 with Ultracel (30 kDa, 0.005 $m^2$, Merck Millipore) to remove low-molecular-weight impurities. The purified solution was filtered through the Millex GV Syringe filter unit (0.22 μm, Φ 33mm, Merck Millipore). Thus, a D-PBS(-) solution of ADC16 was obtained.

Antibody concentration: 7.91 mg/ml, the average number of drugs bound to an antibody molecule (n): 3.78 (HIC-UV analysis (area% @220 nm): DAR0 (6.85%), DAR2 (27.11%), DAR3 (2.47%), DAR4 (37.59%), DAR5 (2.61%), DAR6 (17.04%), DAR8 (6.31%))

[Production Example 17: Antibody-drug conjugate 17 (ADC17)]

Antibody 10 prepared in Example 1 (12.02 mg/ml, 5.0 ml) was diluted with the addition of 2.0 ml of D-PBS(-). While agitating the solution at room temperature, 812.18 μl of a solution of TCEP ▪ HCl in 5 mM D-PBS(-) was added to the antibody solution, and the reaction solution was agitated at 37°C for 1 hour. Thereafter, 422.34 μl of a solution of MC-VC-PAB-MMAE in 10 mM DMSO was added thereto, and the resultant was agitated at room temperature for an additional 1 hour.

Gel filtration was performed with the use of the Zeba Spin Desalting Columns (40 K MWCO, 10 ml, 6 columns, ThermoScientific) to remove low-molecular-weight impurities from the reaction solution. The column eluates obtained were integrated and concentrated to a volume of approximately 6 ml with the use of Amicon Ultra-15 (10 K MWCO, Merck Millipore). The concentrate was purified again with the use of the Zeba Spin Desalting Columns. The column eluates obtained were integrated and filtered through the Millex GV Syringe filter unit (0.22 μm, Φ 33mm, Merck Millipore). Thus, a D-PBS(-) solution of ADC17 was obtained.

Antibody concentration: 5.61 mg/ml, the average number of drugs bound to an antibody molecule (n): 7.91 (HIC-UV analysis (area % at 220 nm): DAR6 (3.30%), DAR8 (96.34%))

[Example 9: Cytotoxicity mediated by ADC]

The cytotoxicity of ADC15, ADC16, and ADC17 against the pancreatic cancer cell line BxPC-3 and the human T

cell leukemia cell line Jurkat was evaluated by the CellTiter-Glo 2.0 luminescent cell viability assay (Promega). The BxPC-3 cells and the Jurkat cells were seeded at 350 cells/well and 150 cells/well, respectively, on a 384-well plate, ADC16 and ADC17 were added, and culture was performed at 37°C for 5 days. Thereafter, the reagent for CellTiter-Glo 2.0 luminescent cell viability assay was added. The relative luminescence was measured using the EnSpire Multimode Plate Reader (PerkinElmer), the data were analyzed using the 4 Parameter Logistic Model, and $IC_{50}$ for each cancer cell line was determined. The results are shown in Table 7. While ADC16 and ADC17 exerted the cytotoxicity against the CD138-positive BxPC-3 cells, ADC16 and ADC17 did not exert the cytotoxicity against the CD138-negative Jurkat cells. This indicates that ADC16 and ADC17 exert the cytotoxicity by the antigen-dependent mechanism. The antibody, the linker, and the payload of ADC16 are common with those of ADC17, although the number of drugs bound to an antibody molecule is different (ADC16: 3.78, ADC17: 7.91). ADC16 and ADC17 are considered to maintain the antigen-dependent cytotoxicity.

[Table 7]

| | $IC_{50}$ (ng/ml) | |
|---|---|---|
| | BxPC-3 | Jurkat |
| | (CD138-positive) | (CD138-negative) |
| ADC16 | 40 | >8000 |
| ADC17 | 9 | 2791 |

[Example 10: Evaluation of ADC stability]

The concentration of ADC15 was adjusted to approximately 2.71 mg/ml with phosphate-buffered saline (Dulbecco's PBS; pH 7.4), that of ADC16 was adjusted to 7.91 mg/ml therewith, and that of ADC17 was adjusted to 5.61 mg/ml therewith. The denaturation midpoint temperature, the aggregation initiation temperature, and the particle size distribution of the antibody solutions were determined using a protein physical property evaluator UNcle (UNCHAINED LABS). Specifically, the denaturation midpoint temperature and the aggregation initiation temperature were determined by simultaneously performing fluorescent spectral measurement and static light scatter measurement while raising the temperature of the antibodies from 25°C to 95°C by 1°C every minute. On the basis of the results of the fluorescent spectral measurement, the midpoint temperature of peak shift; i.e., the denaturation midpoint temperature, was determined (n = 3) using the phenomenon that the peak center of the fluorescent spectra of amino acids internalized in the antibody would shift upon antibody denaturation. On the basis of the results of the static light scatter measurement, the temperature at which the scattered light at 266 nm becomes intensified; i.e., the aggregation initiation temperature, was determined (n = 3) using the phenomenon that the scattering intensity would be increased upon aggregation.

With the use of ADC15 (2.71 mg/ml), ADC16 (7.91 mg/ml), and ADC17 (5.61 mg/ml) prepared with phosphate-buffered saline (Dulbecco's PBS; pH 7.4), the molecular size was measured every 30 minutes with the elapse of time by dynamic light scattering measurement under storage conditions at 50°C for 48 hours, and a difference between the initial average molecular size and the average molecular size 48 hours after the initiation of the measurement was determined to evaluate the aggregation properties (n = 3).

The results are shown in Table 8.

[Table 8]

| | Denaturation midpoint temperature $T_m2$ (°C) | Aggregation initiation temperature $T_{agg}266$ (°C) | Increase in average molecular size (nm) |
|---|---|---|---|
| ADC15 | 71.4 | 61.1 | 72.5 |
| ADC16 | 84.0 | 75.3 | 5.7 |
| ADC17 | 82.8 | 75.7 | 3.2 |

ADC16 and ADC17 were found to have a higher denaturation midpoint temperature and a higher aggregation initiation temperature than those of the ADC15 described in Patent Literature 1. The denaturation midpoint temperature and the aggregation initiation temperature are known to be correlated with long-term storage stability, and the results demonstrate high stability of the ADC of the present invention.

As a result of storage at 50°C for 48 hours, ADC16 and ADC17 exhibited smaller increases in the average molecular size, compared with those of the ADC15 described in Patent Literature 1. This indicates low-aggregation

properties of the ADC of the present invention.

A similar test was performed with the use of antibodies by themselves. As a result, Antibody 10 to Antibody 12 were found to exhibit superior stability to that of Antibody 1 and Antibody 2 as with the results demonstrated above.

As described above, the antibody and the ADC of the present invention have excellent stability upon heat application and, accordingly, the antibody and the ADC are useful as pharmaceutical preparations or starting materials thereof.

Sequence Listing Free Text

SEQ ID NO: 1: the amino acid sequence of CDR1 in the heavy chain of Antibody 1 to Antibody 12;
SEQ ID NO: 2: the amino acid sequence of CDR2 in the heavy chain of Antibody 1 to Antibody 12;
SEQ ID NO: 3: the amino acid sequence of CDR3 in the heavy chain of Antibody 1 to Antibody 12;
SEQ ID NO: 4: the amino acid sequence of CDR1 in the light chain of Antibody 1 to Antibody 12;
SEQ ID NO: 5: the amino acid sequence of CDR2 in the light chain of Antibody 1 to Antibody 12;
SEQ ID NO: 6: the amino acid sequence of CDR3 in the light chain of Antibody 1 to Antibody 12;
SEQ ID NO: 7: the nucleotide sequence of cDNA encoding a variable region of the heavy chain of Antibody 1;
SEQ ID NO: 8: the amino acid sequence of a variable region of the heavy chain of Antibody 1;
SEQ ID NO: 9: the nucleotide sequence of cDNA encoding a variable region of the light chain of Antibody 1 and Antibody 2;
SEQ ID NO: 10: the amino acid sequence of a variable region of the light chain of Antibody 1 and Antibody 2;
SEQ ID NO: 11: the nucleotide sequence of cDNA encoding a variable region of the heavy chain of Antibody 2;
SEQ ID NO: 12: the amino acid sequence of a variable region of the heavy chain of Antibody 2;
SEQ ID NO: 13: the nucleotide sequence of cDNA encoding a variable region of the heavy chain of Antibody 3 and Antibody 10;
SEQ ID NO: 14: the amino acid sequence of a variable region of the heavy chain of Antibody 3 and Antibody 10;
SEQ ID NO: 15: the nucleotide sequence of cDNA encoding a variable region of the light chain of Antibody 3 to Antibody 6 and Antibody 10 to Antibody 12;
SEQ ID NO: 16: the amino acid sequence of a variable region of the light chain of Antibody 3 to Antibody 6 and Antibody 10 to Antibody 12;
SEQ ID NO: 17: the nucleotide sequence of cDNA encoding a variable region of the heavy chain of Antibody 4, Antibody 7, and Antibody 11;
SEQ ID NO: 18: the amino acid sequence of a variable region of the heavy chain of Antibody 4, Antibody 7, and Antibody 11;
SEQ ID NO: 19: the nucleotide sequence of cDNA encoding a variable region of the heavy chain of Antibody 5 and of Antibody 8;
SEQ ID NO: 20: the amino acid sequence of a variable region of the heavy chain of Antibody 5 and Antibody 8;
SEQ ID NO: 21: the nucleotide sequence of cDNA encoding a variable region of the heavy chain of Antibody 6, Antibody 9, and Antibody 12;
SEQ ID NO: 22: the amino acid sequence of a variable region of the heavy chain of Antibody 6, Antibody 9, and Antibody 12;
SEQ ID NO: 23: the nucleotide sequence of cDNA encoding a variable region of the light chain of Antibody 7 to Antibody 9;
SEQ ID NO: 24: the amino acid sequence of a variable region of the light chain of Antibody 7 to Antibody 9;
SEQ ID NO: 25: the full-length nucleotide sequence of the heavy chain of Antibody 1;
SEQ ID NO: 26: the full-length amino acid sequence of the heavy chain of Antibody 1;
SEQ ID NO: 27: the full-length nucleotide sequence of the light chain of Antibody 1 and Antibody 2;
SEQ ID NO: 28: the full-length amino acid sequence of the light chain of Antibody 1 and Antibody 2;
SEQ ID NO: 29: the full-length nucleotide sequence of the heavy chain of Antibody 2;
SEQ ID NO: 30: the full-length amino acid sequence of the heavy chain of Antibody 2;
SEQ ID NO: 31: the full-length nucleotide sequence of the heavy chain of Antibody 3;
SEQ ID NO: 32: the full-length amino acid sequence of the heavy chain of Antibody 3;
SEQ ID NO: 33: the full-length nucleotide sequence of the light chain of Antibody 3 to Antibody 6 and Antibody 10 to Antibody 12;
SEQ ID NO: 34: the full-length amino acid sequence of the light chain of Antibody 3 to Antibody 6 and Antibody 10 to Antibody 12;
SEQ ID NO: 35: the full-length nucleotide sequence of the heavy chain of Antibody 4 and Antibody 7;

SEQ ID NO: 36: the full-length amino acid sequence of the heavy chain of Antibody 4 and Antibody 7;
SEQ ID NO: 37: the full-length nucleotide sequence of the heavy chain of Antibody 5 and Antibody 8;
SEQ ID NO: 38: the full-length amino acid sequence of the heavy chain of Antibody 5 and Antibody 8;
SEQ ID NO: 39: the full-length nucleotide sequence of the heavy chain of Antibody 6 and Antibody 9;
SEQ ID NO: 40: the full-length amino acid sequence of the heavy chain of Antibody 6 and Antibody 9;
SEQ ID NO: 41: the full-length nucleotide sequence of the light chain of Antibody 7 to Antibody 9;
SEQ ID NO: 42: the full-length amino acid sequence of the light chain of Antibody 7 to Antibody 9;
SEQ ID NO: 43: the full-length nucleotide sequence of the heavy chain of Antibody 10;
SEQ ID NO: 44: the full-length amino acid sequence of the heavy chain of Antibody 10;
SEQ ID NO: 45: the full-length nucleotide sequence of the heavy chain of Antibody 11;
SEQ ID NO: 46: the full-length amino acid sequence of the heavy chain of Antibody 11;
SEQ ID NO: 47: the full-length nucleotide sequence of the heavy chain of Antibody 12;
SEQ ID NO: 48: the full-length amino acid sequence of the heavy chain of Antibody 12;
SEQ ID NO: 49: the amino acid sequence of the human CD138 protein; and
SEQ ID NO: 50: a particular epitope sequence in CD138.

All publications, patents, and patent applications cited herein are incorporated herein by reference in their entirety.

## Claims

1. An antibody-drug conjugate (ADC) represented by Formula 1:

Ab-(L-D)p (1)

wherein Ab represents an antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16,

wherein

L represents a linker;
D represents monomethyl auristatin; and
p is 1 to 12.

2. The antibody-drug conjugate (ADC) according to claim 1, wherein Ab represents an antibody selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence

represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138.

**3.** The antibody-drug conjugate (ADC) according to claim 1 or 2, wherein Ab represents an antibody selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34.

**4.** The antibody-drug conjugate (ADC) according to any one of claims 1 to 3, wherein L represents 6-maleimidocaproyl-valine-citrulline-p-aminobenzyloxycarbonyl (MC-Val-Cit-PAB).

**5.** The antibody-drug conjugate (ADC) according to any one of claims 1 to 4, wherein D represents monomethyl auristatin E.

**6.** The antibody-drug conjugate (ADC) according to any one of claims 1 to 5, wherein p is 1 to 8.

**7.** The antibody-drug conjugate (ADC) according to any one of claims 1 to 6, wherein

Ab represents an antibody comprising a heavy chain comprising a variable region consisting of the amino acid sequence represented by SEQ ID NO: 14 and a light chain comprising a variable region consisting of the amino acid sequence represented by SEQ ID NO: 16,
L represents 6-maleimidocaproyl-valine-citrulline-p-aminobenzyloxycarbonyl (MC-Val-Cit-PAB),
D represents monomethyl auristatin E, and
P is 1 to 8.

**8.** The antibody-drug conjugate (ADC) according to any one of claims 1 to 7,
wherein

Ab represents an antibody comprising a heavy chain consisting of the amino acid sequence represented by SEQ ID NO: 44 and a light chain consisting of the amino acid sequence represented by SEQ ID NO: 34,
L represents 6-maleimidocaproyl-valine-citrulline-p-aminobenzyloxycarbonyl (MC-Val-Cit-PAB),
D represents monomethyl auristatin E, and
P is 1 to 8.

**9.** A pharmaceutical composition comprising the antibody-drug conjugate (ADC) according to any one of claims 1 to 8 and a carrier.

**10.** An anti-tumor agent comprising the antibody-drug conjugate (ADC) according to any one of claims 1 to 8.

**11.** The anti-tumor agent according to claim 10, which is used for treating cancer that expresses CD138.

**12.** The anti-tumor agent according to claim 10, which is administered in combination with another anti-tumor agent.

**13.** The anti-tumor agent according to claim 10, which is used for treatment of gastric cancer, pancreatic cancer, large

bowel cancer, lung cancer, breast cancer, or urothelial cancer.

**14.** An antibody selected from the group consisting of (1) to (3) below that binds specifically to CD138 and has a human IgG1 constant region:

(1) an antibody comprising a heavy chain comprising a variable region consisting of the amino acid sequence represented by SEQ ID NO: 14 and a light chain comprising a variable region consisting of the amino acid sequence represented by SEQ ID NO: 16;
(2) an antibody comprising a heavy chain comprising a variable region consisting of the amino acid sequence represented by SEQ ID NO: 18 and a light chain comprising a variable region consisting of the amino acid sequence represented by SEQ ID NO: 16; and
(3) an antibody comprising a heavy chain comprising a variable region consisting of the amino acid sequence represented by SEQ ID NO: 22 and a light chain comprising a variable region consisting of the amino acid sequence represented by SEQ ID NO: 16.

**15.** The antibody according to claim 14, which is selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34, or
an antibody comprising a heavy chain comprising a variable region consisting of the amino acid sequence represented by SEQ ID NO: 14 and having at least 98% sequence identity to the amino acid sequence represented by SEQ ID NO: 44 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138;
(2) an antibody comprising a heavy chain consisting of the amino acid sequence represented by SEQ ID NO: 46 and a light chain consisting of the amino acid sequence represented by SEQ ID NO: 34, or
an antibody comprising a heavy chain comprising a variable region consisting of the amino acid sequence represented by SEQ ID NO: 18 and having at least 98% sequence identity to the amino acid sequence represented by SEQ ID NO: 46 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138; and
(3) an antibody comprising a heavy chain consisting of the amino acid sequence represented by SEQ ID NO: 48 and a light chain consisting of the amino acid sequence represented by SEQ ID NO: 34, or
an antibody comprising a heavy chain comprising a variable region consisting of the amino acid sequence represented by SEQ ID NO: 22 and having at least 98% sequence identity to the amino acid sequence represented by SEQ ID NO: 48 and a light chain comprising a variable region consisting of the amino acid sequence represented by SEQ ID NO: 16 and having at least 98% sequence identity to the amino acid sequence represented by SEQ ID NO: 34 and having the capability of binding specifically to CD138.

**16.** The antibody according to claim 14 or 15, which is selected from the group consisting of (1) to (3) below:

(1) an antibody comprising a heavy chain consisting of the amino acid sequence represented by SEQ ID NO: 44 and a light chain consisting of the amino acid sequence represented by SEQ ID NO: 34;
(2) an antibody comprising a heavy chain consisting of the amino acid sequence represented by SEQ ID NO: 46 and a light chain consisting of the amino acid sequence represented by SEQ ID NO: 34; and
(3) an antibody comprising a heavy chain consisting of the amino acid sequence represented by SEQ ID NO: 48 and a light chain consisting of the amino acid sequence represented by SEQ ID NO: 34.

**17.** A method for treating cancer comprising a step of administering the antibody-drug conjugate (ADC) according to any one of claims 1 to 8 to a subject.

**18.** Use of the antibody-drug conjugate (ADC) according to any one of claims 1 to 8 for treating cancer.

**19.** The antibody-drug conjugate (ADC) according to any one of claims 1 to 8, which is used for treating cancer.

**20.** Use of the antibody-drug conjugate (ADC) according to any one of claims 1 to 8 in the manufacture of an anti-tumor agent.

**21.** A method for treating cancer comprising a step of administering the antibody-drug conjugate (ADC) according to any one of claims 1 to 8 and another anti-tumor agent to a subject.

**22.** Use of the antibody-drug conjugate (ADC) according to any one of claims 1 to 8 for treating cancer, which is administered in combination with another anti-tumor agent.

**23.** The antibody-drug conjugate (ADC) according to any one of claims 1 to 8 used for treating cancer, which is administered in combination with another anti-tumor agent.

**24.** Use of the antibody-drug conjugate (ADC) according to any one of claims 1 to 8 in the manufacture of an anti-tumor agent, which is administered in combination with another anti-tumor agent.

Fig. 1

A

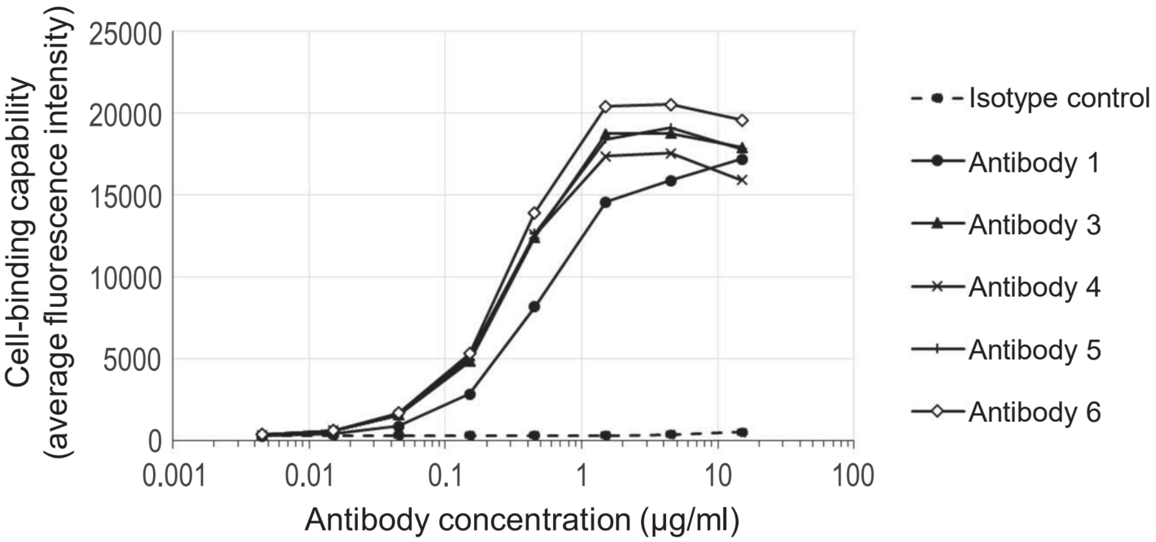
Cell-binding capability
(average fluorescence intensity)
25000
20000
15000
10000
5000
0
0.001
0.01
0.1
1
10
100
Antibody concentration (µg/ml)
Isotype control
Antibody 1
Antibody 3
Antibody 4
Antibody 5
Antibody 6

B

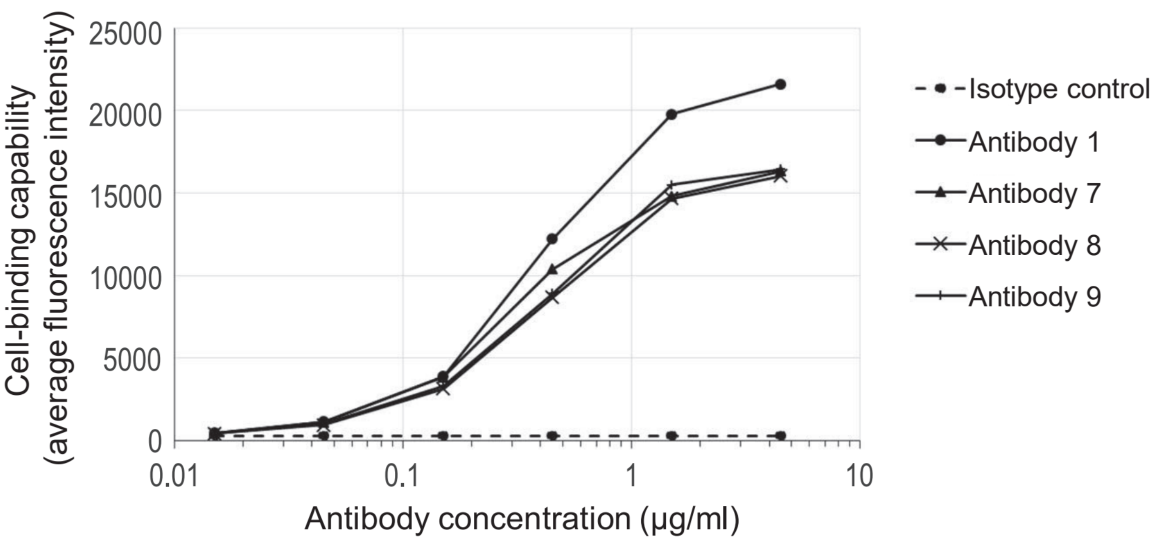
Cell-binding capability
(average fluorescence intensity)
25000
20000
15000
10000
5000
0
0.01
0.1
1
10
Antibody concentration (µg/ml)
Isotype control
Antibody 1
Antibody 7
Antibody 8
Antibody 9

# Fig. 2-1

| | Antibody | Name and structure of linker-payload | |
|---|---|---|---|
| ADC1 | Antibody 1 | SMCC-DM1 | 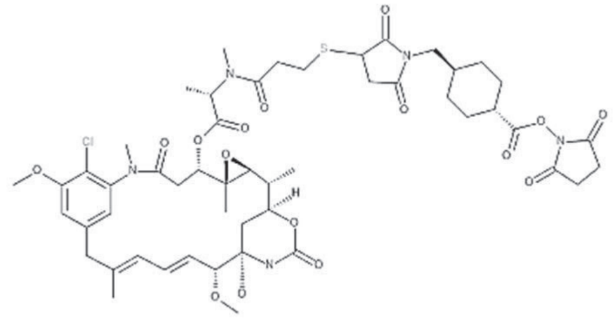 |
| ADC2 | Antibody 2 | SPDB-DM4 | 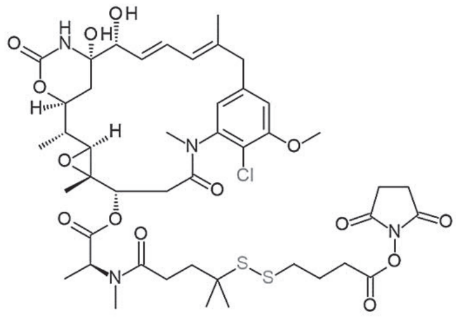 |
| ADC3 | Antibody 1 | MC-VC-PAB-MMAE | 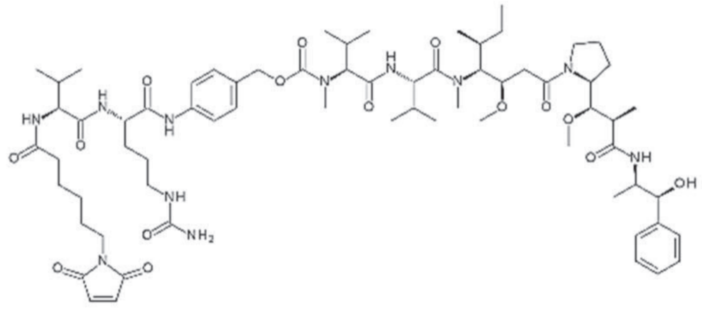 |
| ADC4 | Antibody 1 | MC-Val-Cit-PAB-MMAF | 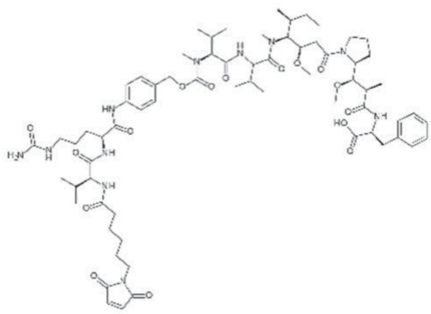 |
| ADC5 | Antibody 1 | MC-GGFG-Dxd | 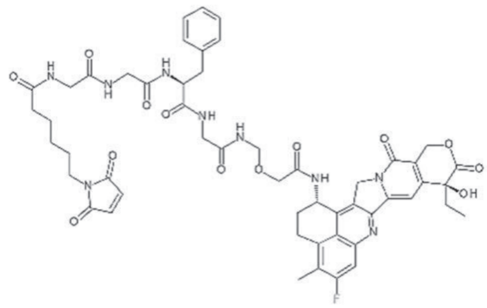 |

# Fig. 2-2

| | Antibody | Name and structure of linker-payload | |
|---|---|---|---|
| ADC6 | Antibody 1 | MC-GGFG-Dx8951 | 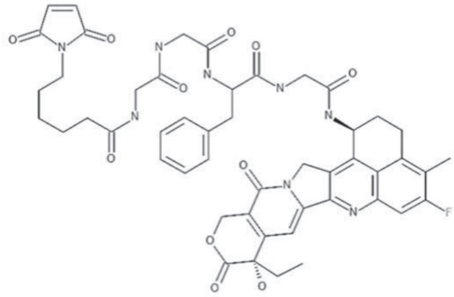 |
| ADC7 | Antibody 1 | MC-Val-Ala-PBD | 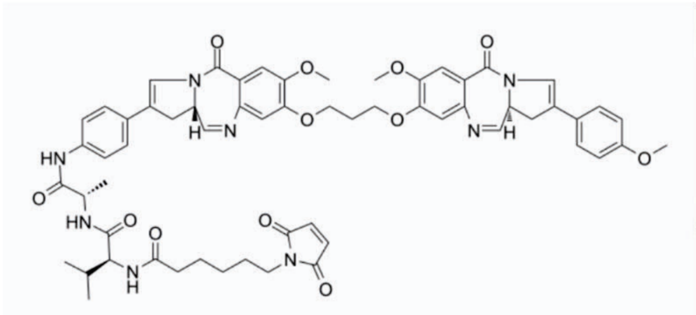 |
| ADC8 | Antibody 3 | MC-VC-PAB-MMAE | 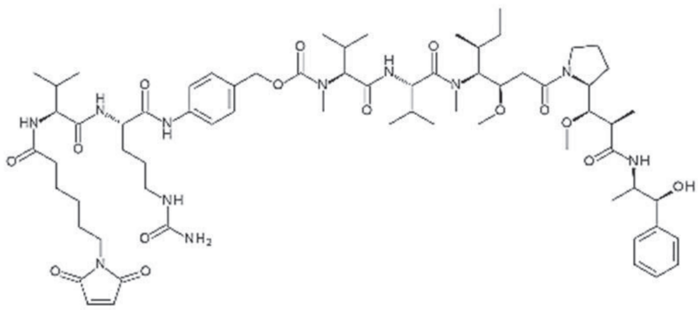 |
| ADC9 | Antibody 4 | MC-VC-PAB-MMAE | 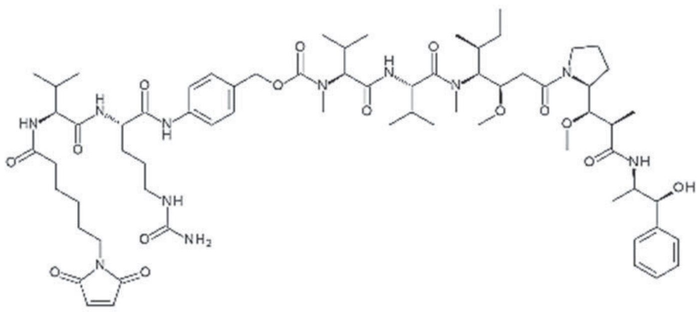 |
| ADC10 | Antibody 5 | MC-VC-PAB-MMAE | 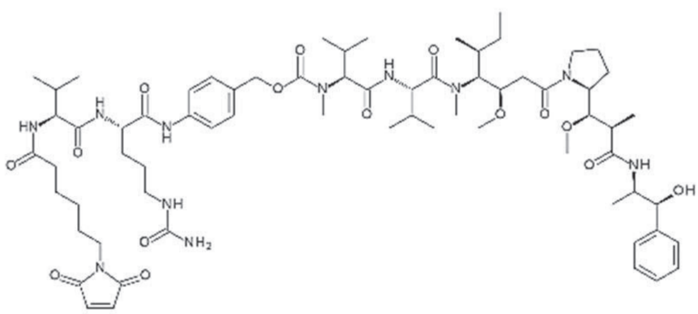 |

Fig. 2-3

| | Antibody | Name and structure of linker-payload | |
|---|---|---|---|
| ADC11 | Antibody 6 | MC-VC-PAB-MMAE | 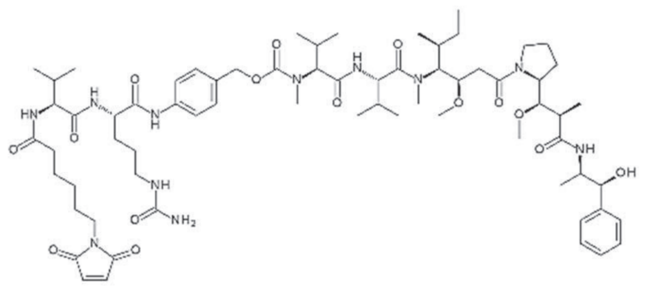 |
| ADC12 | Antibody 10 | MC-VC-PAB-MMAE | 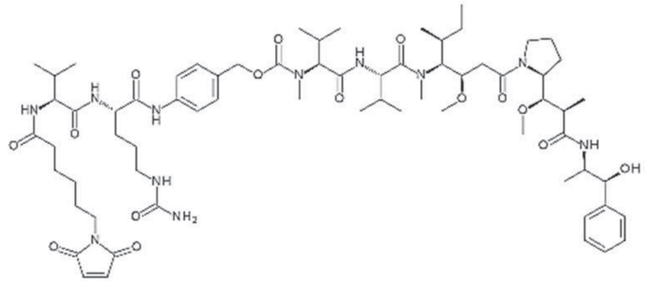 |
| ADC13 | Antibody 11 | MC-VC-PAB-MMAE | 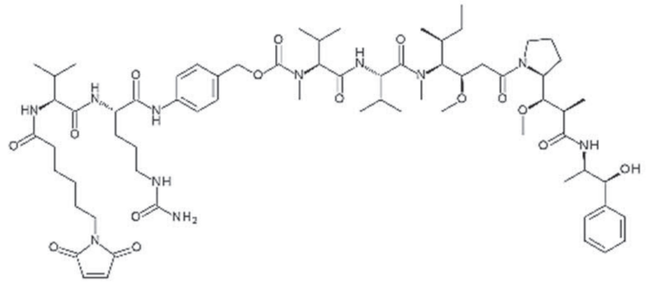 |
| ADC14 | Antibody 12 | MC-VC-PAB-MMAE | 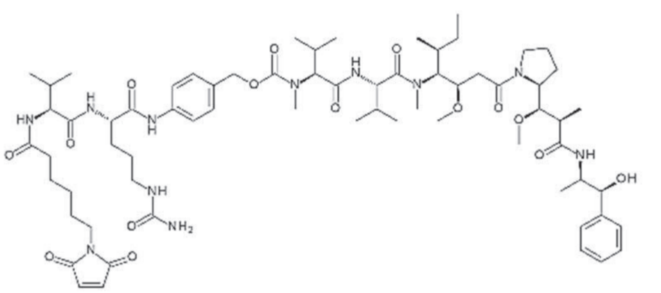 |

Fig. 3

A

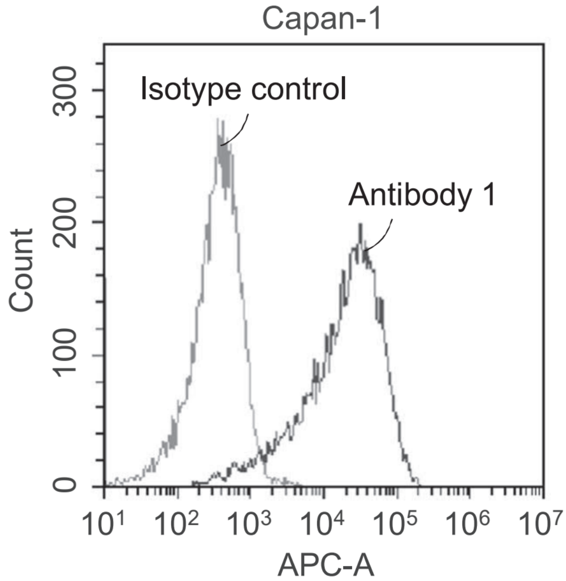
Capan-1
Isotype control
Antibody 1
Count
300
200
100
0
10¹ 10² 10³ 10⁴ 10⁵ 10⁶ 10⁷
APC-A

B

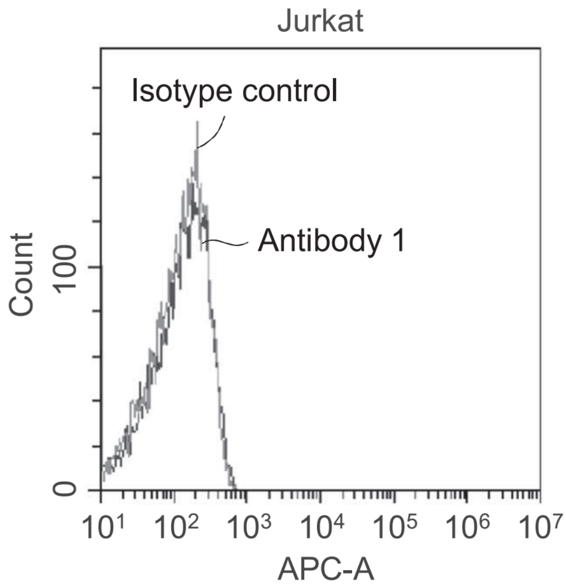
Jurkat
Isotype control
Antibody 1
Count
100
0
10¹ 10² 10³ 10⁴ 10⁵ 10⁶ 10⁷
APC-A

Fig. 4

A

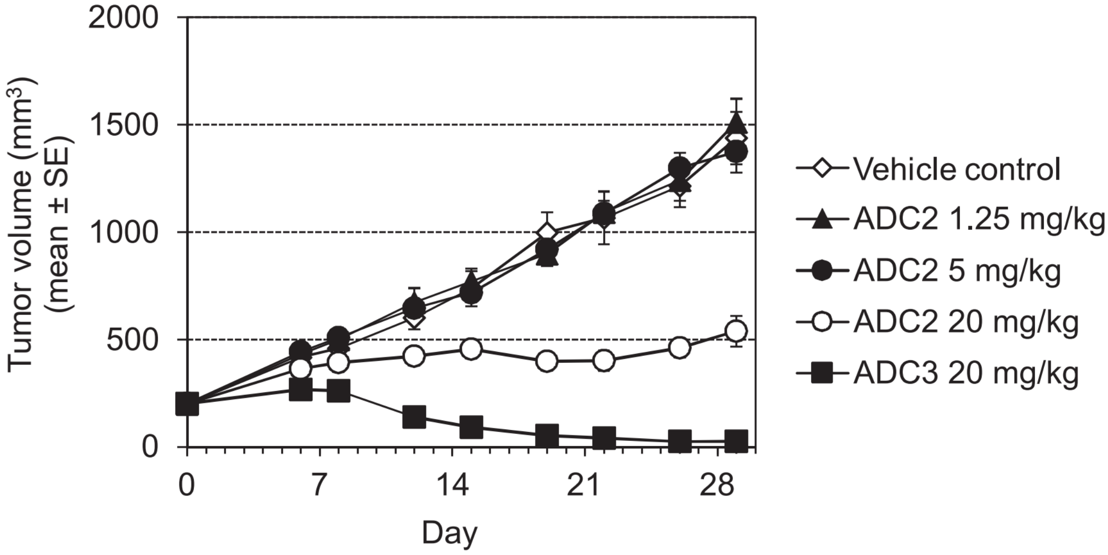
Tumor volume (mm³)
(mean ± SE)
2000
1500
1000
500
0
0
7
14
21
28
Day
Vehicle control
ADC2 1.25 mg/kg
ADC2 5 mg/kg
ADC2 20 mg/kg
ADC3 20 mg/kg

B

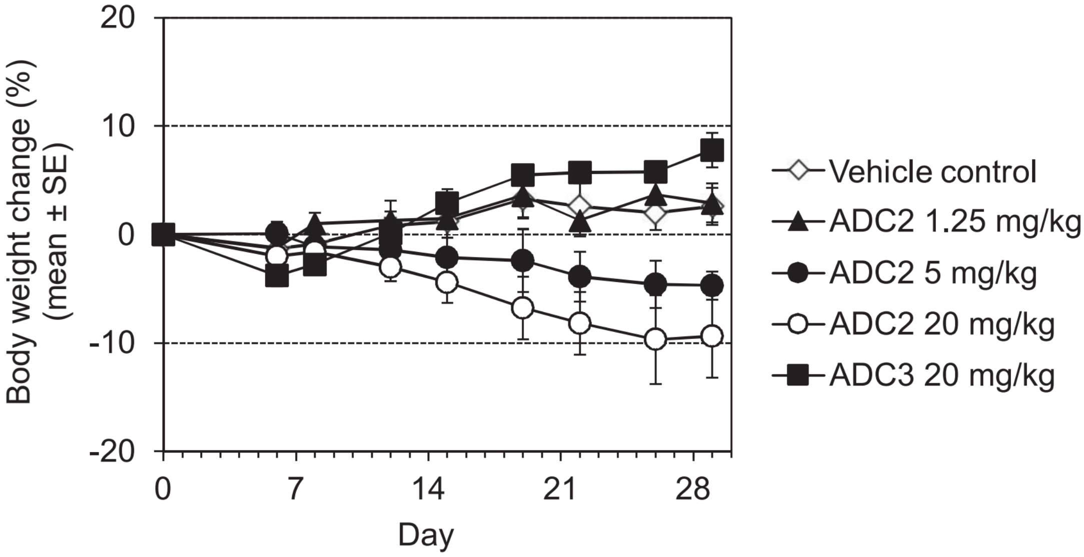
Body weight change (%)
(mean ± SE)
20
10
0
-10
-20
0
7
14
21
28
Day
Vehicle control
ADC2 1.25 mg/kg
ADC2 5 mg/kg
ADC2 20 mg/kg
ADC3 20 mg/kg

Fig. 5

A

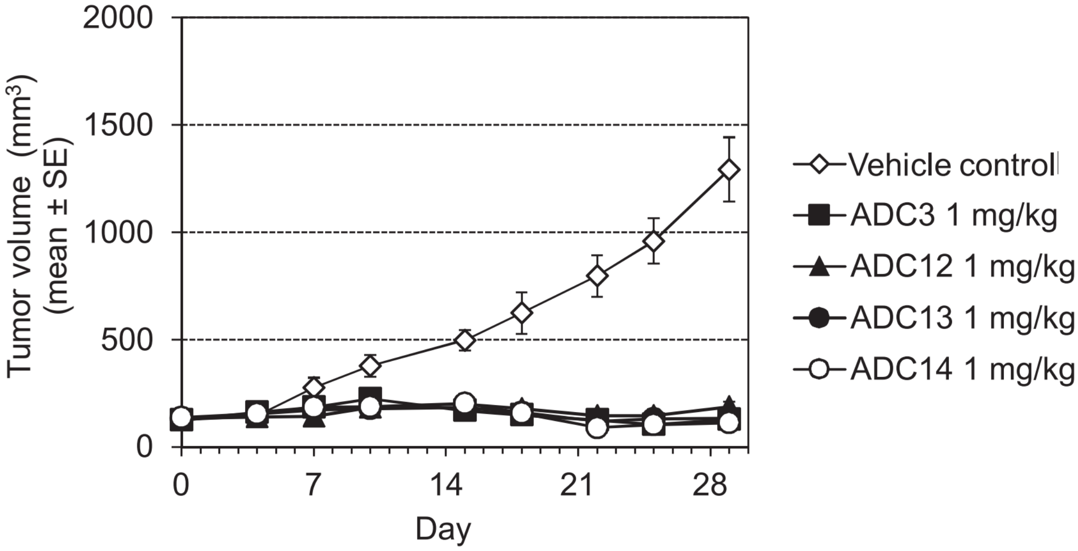
2000
1500
1000
500
0
Tumor volume (mm³) (mean ± SE)
0
7
14
21
28
Day
Vehicle controll
ADC3 1 mg/kg
ADC12 1 mg/kg
ADC13 1 mg/kg
ADC14 1 mg/kg

B

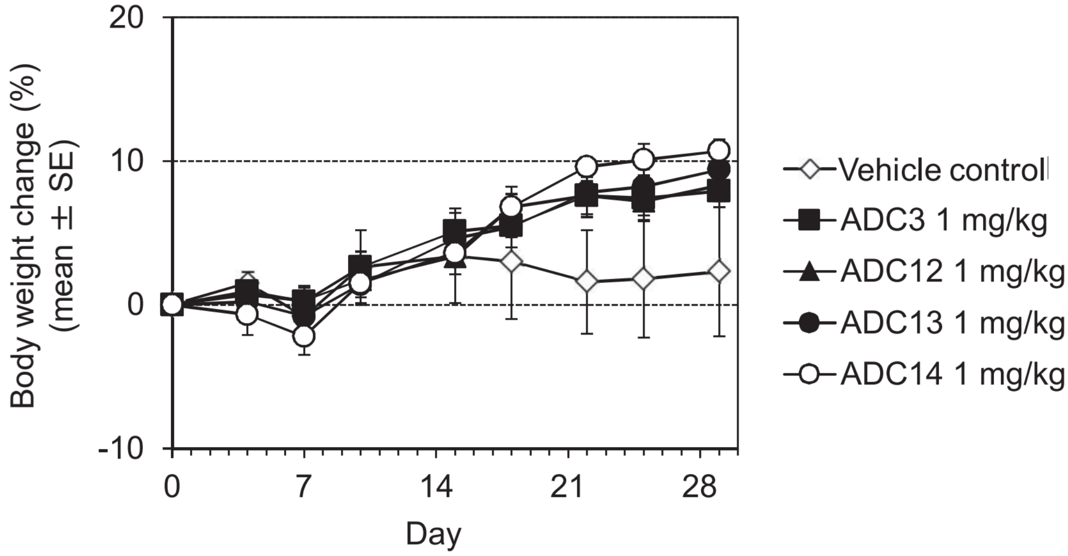
20
10
0
-10
Body weight change (%) (mean ± SE)
0
7
14
21
28
Day
Vehicle controll
ADC3 1 mg/kg
ADC12 1 mg/kg
ADC13 1 mg/kg
ADC14 1 mg/kg

# Fig. 6

SEQ ID NO: 1
<223>  Amino acid sequence of CDR1 of the heavy chain from Antibody 1 to Antibody 12
NYWIE

SEQ ID NO: 2
<223>  Amino acid sequence of CDR2 of the heavy chain from Antibody 1 to Antibody 12
EILPGTGRTIYNEKFKG

SEQ ID NO: 3
<223>  Amino acid sequence of CDR3 of the heavy chain from Antibody 1 to Antibody 12
RDYYGNFYYAMDY

SEQ ID NO: 4
<223>  Amino acid sequence of CDR1 of the light chain from Antibody 1 to Antibody 12
SASQGINNYLN

SEQ ID NO: 5
<223>  Amino acid sequence of CDR2 of the light chain from Antibody 1 to Antibody 12
YTSTLQS

SEQ ID NO: 6
<223>  Amino acid sequence of CDR3 of the light chain from Antibody 1 to Antibody 12
QQYSKLPRT

SEQ ID NO: 7
<223>  Nucleotide sequence of cDNA encoding the variable region of the heavy chain of Antibody 1
CAGGTTCAGCTGCAGCAGTCTGGCAGCGAGCTTATGATGCCTGGCGCCTCCGTGAAGATCAG
CTGTAAAGCCACCGGCTACACCTTCAGCAACTACTGGATCGAGTGGGTCAAGCAGAGGCCTG
GCCATGGACTGGAATGGATCGGAGAGATCCTGCCTGGCACCGGCAGAACCATCTACAACGAG
AAGTTCAAGGGCAAAGCCACCTTCACCGCCGACATCAGCAGCAACACAGTGCAGATGCAGCT
GAGCAGCCTGACCAGCGAAGATAGCGCCGTGTACTACTGCGCCAGACGGGACTACTACGGCA
ACTTCTACTACGCCATGGATTACTGGGGCCAGGGCACCCTGGTTACAGTGTCTAGC

# Fig. 7

SEQ ID NO: 8
<223> Amino acid sequence of the variable region of the heavy chain of Antibody 1
QVQLQQSGSELMMPGASVKISCKATGYTFSNYWIEWVKQRPGHGLEWIGEILPGTGRTIYNE
KFKGKATFTADISSNTVQMQLSSLTSEDSAVYYCARRDYYGNFYYAMDYWGQGTLVTVSS

SEQ ID NO: 9
<223> Nucleotide sequence of cDNA encoding the variable region of the light chain of Antibody 1 and Antibody 2
GACATCCAGATGACCCAGAGCACCAGCAGCCTGTCTGCCTCTCTGGGCGATAGAGTGACCAT
CAGCTGTAGCGCCAGCCAGGGCATCAACAACTACCTGAACTGGTATCAGCAGAAACCCGACG
GCACCGTGGAACTGCTGATCTACTACACCAGCACACTGCAGAGCGGCGTGCCCAGCAGATTT
TCTGGCTCTGGCAGCGGCACCGACTACAGCCTGACAATCAGCAACCTGGAACCTGAGGACAT
CGGCACCTACTACTGCCAGCAGTACAGCAAGCTGCCCAGAACCTTTGGCGGAGGCACCAAGC
TGGAAATCAAG

SEQ ID NO: 10
<223> Amino acid sequence of the variable region of the light chain of Antibody 1 and Antibody 2
DIQMTQSTSSLSASLGDRVTISCSASQGINNYLNWYQQKPDGTVELLIYYTSTLQSGVPSRF
SGSGSGTDYSLTISNLEPEDIGTYYCQQYSKLPRTFGGGTKLEIK

SEQ ID NO: 11
<223> Nucleotide sequence of cDNA encoding the variable region of the heavy chain of Antibody 2
CAGGTTCAGCTGCAGCAGTCTGGCAGCGAGCTTATGATGCCTGGCGCCTCCGTGAAGATCAG
CTGTAAAGCCACCGGCTACACCTTCAGCAACTACTGGATCGAGTGGGTCAAGCAGAGGCCTG
GCCATGGACTGGAATGGATCGGAGAGATCCTGCCTGGCACCGGCAGAACCATCTACAACGAG
AAGTTCAAGGGCAAAGCCACCTTCACCGCCGACATCAGCAGCAACACAGTGCAGATGCAGCT
GAGCAGCCTGACCAGCGAAGATAGCGCCGTGTACTACTGCGCCAGACGGGACTACTACGGCA
ACTTCTACTACGCCATGGATTACTGGGGCCAGGGCACCAGCGTGACAGTGTCTAGC

SEQ ID NO: 12
<223> Amino acid sequence of the variable region of the heavy chain of Antibody 2
QVQLQQSGSELMMPGASVKISCKATGYTFSNYWIEWVKQRPGHGLEWIGEILPGTGRTIYNE
KFKGKATFTADISSNTVQMQLSSLTSEDSAVYYCARRDYYGNFYYAMDYWGQGTSVTVSS

# Fig. 8

SEQ ID NO: 13
<223> Nucleotide sequence of cDNA encoding the variable region of the heavy chain of Antibody 3 and Antibody 10
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGCCACCTTCACCGCCGACATCTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCC

SEQ ID NO: 14
<223> Amino acid sequence of the variable region of the heavy chain of Antibody 3 and Antibody 10
QVQLVQSGAEVKKPGSSVKVSCKASGYTFSNYWIEWVRQAPGQGLEWIGEILPGTGRTIYNE
KFKGRATFTADISTSTAYMELSSLRSEDTAVYYCARRDYYGNFYYAMDYWGQGTLVTVSS

SEQ ID NO: 15
<223> Nucleotide sequence of cDNA encoding the variable region of the light chain of Antibodies 3, 4, 5, 6, 10, 11, and 12
GACATCCAGATGACCCAGTCCCCCTCCTCCCTCTCCGCCTCCGTCGGCGACCGCGTCACCAT
CACCTGCTCCGCCTCCCAGGGCATCAACAACTACCTCAACTGGTACCAGCAGAAGCCCGGCA
AGGCCCCCAAGCTCCTCATCTACTACACCTCCACCCTCCAGTCCGGCGTCCCCTCCCGCTTC
TCCGGCTCCGGCTCCGGCACCGACTACACCCTCACCATCTCCTCCCTCCAGCCCGAGGACTT
CGCCACCTACTACTGCCAGCAGTACTCCAAGCTCCCCCGCACCTTCGGCGGCGGCACCAAGG
TCGAGATCAAG

SEQ ID NO: 16
<223> Amino acid sequence of the variable region of the light chain of Antibodies 3, 4, 5, 6, 10, 11, and 12
DIQMTQSPSSLSASVGDRVTITCSASQGINNYLNWYQQKPGKAPKLLIYYTSTLQSGVPSRF
SGSGSGTDYTLTISSLQPEDFATYYCQQYSKLPRTFGGGTKVEIK

SEQ ID NO: 17
<223> Nucleotide sequence of cDNA encoding the variable region of the heavy chain of Antibodies 4, 7, and 11
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGTCACCTTCACCGCCGACATCTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCC

# Fig. 9

SEQ ID NO: 18
<223> Amino acid sequence of the variable region of the heavy chain of Antibodies 4, 7, and 11
QVQLVQSGAEVKKPGSSVKVSCKASGYTFSNYWIEWVRQAPGQGLEWIGEILPGTGRTIYNE
KFKGRVTFTADISTSTAYMELSSLRSEDTAVYYCARRDYYGNFYYAMDYWGQGTLVTVSS

SEQ ID NO: 19
<223> Nucleotide sequence of cDNA encoding the variable region of the heavy chain of Antibody 5 and Antibody 8
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGCCACCTTCACCGCCGACGAGTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCC

SEQ ID NO: 20
<223> Amino acid sequence of the variable region of the heavy chain of Antibody 5 and Antibody 8
QVQLVQSGAEVKKPGSSVKVSCKASGYTFSNYWIEWVRQAPGQGLEWIGEILPGTGRTIYNE
KFKGRATFTADESTSTAYMELSSLRSEDTAVYYCARRDYYGNFYYAMDYWGQGTLVTVSS

SEQ ID NO: 21
<223> Nucleotide sequence of cDNA encoding the variable region of the heavy chain of Antibodies 6, 9, and 12
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGTCACCTTCACCGCCGACGAGTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCC

SEQ ID NO: 22
<223> Amino acid sequence of the variable region of the heavy chain of Antibodies 6, 9, and 12
QVQLVQSGAEVKKPGSSVKVSCKASGYTFSNYWIEWVRQAPGQGLEWIGEILPGTGRTIYNE
KFKGRVTFTADESTSTAYMELSSLRSEDTAVYYCARRDYYGNFYYAMDYWGQGTLVTVSS

# Fig. 10

SEQ ID NO: 23
<223> Nucleotide sequence of cDNA encoding the variable region of the light chain of Antibodies 7, 8, and 9
GACATCCAGATGACCCAGTCCCCCTCCTCCCTCTCCGCCTCCGTCGGCGACCGCGTCACCAT
CACCTGCTCCGCCTCCCAGGGCATCAACAACTACCTCAACTGGTACCAGCAGAAGCCCGGCA
AGGCCCCCAAGCTCCTCATCTACTACACCTCCACCCTCCAGTCCGGCGTCCCCTCCCGCTTC
TCCGGCTCCGGCTCCGGCACCGACTTCACCCTCACCATCTCCTCCCTCCAGCCCGAGGACTT
CGCCACCTACTACTGCCAGCAGTACTCCAAGCTCCCCCGCACCTTCGGCGGCGGCACCAAGG
TCGAGATCAAG

SEQ ID NO: 24
<223> Amino acid sequence of the variable region of the light chain of Antibodies 7, 8 and 9
DIQMTQSPSSLSASVGDRVTITCSASQGINNYLNWYQQKPGKAPKLLIYYTSTLQSGVPSRF
SGSGSGTDFTLTISSLQPEDFATYYCQQYSKLPRTFGGGTKVEIK

SEQ ID NO: 25
<223> Nucleotide sequence of the heavy chain of Antibody 1
CAGGTTCAGCTGCAGCAGTCTGGCAGCGAGCTTATGATGCCTGGCGCCTCCGTGAAGATCAG
CTGTAAAGCCACCGGCTACACCTTCAGCAACTACTGGATCGAGTGGGTCAAGCAGAGGCCTG
GCCATGGACTGGAATGGATCGGAGAGATCCTGCCTGGCACCGGCAGAACCATCTACAACGAG
AAGTTCAAGGGCAAAGCCACCTTCACCGCCGACATCAGCAGCAACACAGTGCAGATGCAGCT
GAGCAGCCTGACCAGCGAAGATAGCGCCGTGTACTACTGCGCCAGACGGGACTACTACGGCA
ACTTCTACTACGCCATGGATTACTGGGGCCAGGGCACCCTGGTTACAGTGTCTAGCGCCAGC
ACAAAGGGCCCCTCCGTGTTTCCACTGGCTCCTAGCAGCAAGAGCACAAGCGGAGGAACAGC
CGCTCTGGGCTGTCTGGTCAAGGACTACTTTCCCGAGCCTGTGACCGTGTCCTGGAATTCTG
GCGCTCTGACAAGCGGCGTGCACACCTTTCCAGCTGTGCTGCAAAGCAGCGGCCTGTACTCT
CTGAGCAGCGTGGTCACAGTGCCAAGCTCTAGCCTGGGCACCCAGACCTACATCTGCAATGT
GAACCACAAGCCTAGCAACACCAAGGTGGACAAGAAGGTGGAACCCAAGAGCTGCGACAAGA
CCCACACCTGTCCTCCATGTCCTGCTCCAGAACTGCTCGGCGGACCCTCTGTGTTCCTGTTT
CCTCCAAAGCCTAAGGACACCCTGATGATCAGCAGAACCCCTGAAGTGACCTGCGTGGTGGT
GGATGTGTCCCACGAGGATCCCGAAGTGAAGTTCAATTGGTACGTGGACGGCGTGGAAGTGC
ACAACGCCAAGACCAAGCCTAGAGAGGAACAGTACAACAGCACCTACAGAGTGGTGTCCGTG
CTGACCGTGCTGCACCAGGATTGGCTGAACGGCAAAGAGTACAAGTGCAAGGTGTCCAACAA
GGCCCTGCCTGCTCCTATCGAGAAAACCATCAGCAAGGCCAAGGGCCAGCCTAGGGAACCCC
AGGTTTACACACTGCCTCCAAGCCGGGAAGAGATGACCAAGAACCAGGTGTCCCTGACCTGC
CTCGTGAAGGGCTTCTACCCTTCCGATATCGCCGTGGAATGGGAGAGCAATGGCCAGCCTGA
GAACAACTACAAGACAACCCCTCCTGTGCTGGACAGCGACGGCTCATTCTTCCTGTACAGCA
AGCTGACAGTGGACAAGTCCAGATGGCAGCAGGGCAACGTGTTCTCCTGCAGCGTGATGCAC
GAGGCCCTGCACAACCACTACACCCAGAAGTCCCTGAGCCTGTCTCCTGGC

# Fig. 11

SEQ ID NO: 26
<223>  Amino acid sequence of the heavy chain of Antibody 1
QVQLQQSGSELMMPGASVKISCKATGYTFSNYWIEWVKQRPGHGLEWIGEILPGTGRTIYNE
KFKGKATFTADISSNTVQMQLSSLTSEDSAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

SEQ ID NO: 27
<223>  Nucleotide sequence of the light chain of Antibody 1 and Antibody 2
GACATCCAGATGACCCAGAGCACCAGCAGCCTGTCTGCCTCTCTGGGCGATAGAGTGACCAT
CAGCTGTAGCGCCAGCCAGGGCATCAACAACTACCTGAACTGGTATCAGCAGAAACCCGACG
GCACCGTGGAACTGCTGATCTACTACACCAGCACACTGCAGAGCGGCGTGCCCAGCAGATTT
TCTGGCTCTGGCAGCGGCACCGACTACAGCCTGACAATCAGCAACCTGGAACCTGAGGACAT
CGGCACCTACTACTGCCAGCAGTACAGCAAGCTGCCCAGAACCTTTGGCGGAGGCACCAAGC
TGGAAATCAAGCGGACAGTGGCCGCTCCTAGCGTGTTCATCTTTCCACCTAGCGACGAGCAG
CTGAAGTCTGGCACAGCCTCTGTCGTGTGCCTGCTGAACAACTTCTACCCCAGAGAAGCCAA
GGTGCAGTGGAAGGTGGACAACGCCCTGCAGTCCGGCAATAGCCAAGAGAGCGTGACCGAGC
AGGACTCCAAGGACAGCACCTATAGCCTGAGCAGCACCCTGACACTGAGCAAGGCCGACTAC
GAGAAGCACAAAGTGTACGCCTGCGAAGTGACCCACCAGGGCCTTTCTAGCCCTGTGACCAA
GAGCTTCAACCGGGGCGAGTGT

SEQ ID NO: 28
<223>  Amino acid sequence of the light chain of Antibody 1 and Antibody 2
DIQMTQSTSSLSASLGDRVTISCSASQGINNYLNWYQQKPDGTVELLIYYTSTLQSGVPSRF
SGSGSGTDYSLTISNLEPEDIGTYYCQQYSKLPRTFGGGTKLEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

# Fig. 12

SEQ ID NO: 29
<223> Nucleotide sequence of the heavy chain of Antibody 2
CAGGTTCAGCTGCAGCAGTCTGGCAGCGAGCTTATGATGCCTGGCGCCTCCGTGAAGATCAG
CTGTAAAGCCACCGGCTACACCTTCAGCAACTACTGGATCGAGTGGGTCAAGCAGAGGCCTG
GCCATGGACTGGAATGGATCGGAGAGATCCTGCCTGGCACCGGCAGAACCATCTACAACGAG
AAGTTCAAGGGCAAAGCCACCTTCACCGCCGACATCAGCAGCAACACAGTGCAGATGCAGCT
GAGCAGCCTGACCAGCGAAGATAGCGCCGTGTACTACTGCGCCAGACGGGACTACTACGGCA
ACTTCTACTACGCCATGGATTACTGGGGCCAGGGCACCAGCGTGACAGTGTCTAGCGCCTCT
ACAAAGGGACCCAGCGTGTTCCCTCTGGCTCCTTGTAGCAGAAGCACCAGCGAGTCTACAGC
CGCTCTGGGCTGTCTGGTCAAGGACTACTTTCCCGAGCCTGTGACCGTGTCCTGGAATTCTG
GCGCTCTGACAAGCGGCGTGCACACCTTTCCAGCTGTGCTGCAAAGCAGCGGCCTGTACTCT
CTGAGCAGCGTGGTCACAGTGCCTAGCTCTAGCCTGGGCACCAAGACCTACACCTGTAATGT
GGACCACAAGCCTAGCAACACCAAGGTGGACAAGCGCGTGGAATCTAAGTACGGCCCTCCTT
GTCCTAGCTGCCCCGCTCCTGAATTTCTCGGCGGACCTTCCGTGTTCCTGTTTCCTCCAAAG
CCTAAGGACACCCTGATGATCAGCAGAACCCCTGAAGTGACCTGCGTGGTGGTGGACGTGTC
CCAAGAGGATCCTGAGGTGCAGTTCAATTGGTACGTGGACGGCGTGGAAGTGCACAACGCCA
AGACCAAGCCTAGAGAGGAACAGTTCAACAGCACCTACAGAGTGGTGTCCGTGCTGACCGTG
CTGCACCAGGATTGGCTGAACGGCAAAGAGTACAAGTGCAAGGTGTCCAACAAGGGCCTGCC
TAGCAGCATCGAGAAAACCATCAGCAAGGCCAAGGGCCAGCCAAGAGAACCCCAGGTGTACA
CACTGCCTCCAAGCCAAGAGGAAATGACCAAGAACCAGGTGTCCCTGACCTGCCTCGTGAAG
GGCTTCTACCCTTCCGATATCGCCGTGGAATGGGAGAGCAATGGCCAGCCTGAGAACAACTA
CAAGACAACCCCTCCTGTGCTGGACAGCGACGGCTCATTCTTCCTGTACAGCAGACTGACCG
TGGACAAGAGCAGATGGCAAGAGGGCAACGTGTTCTCCTGCAGCGTGATGCACGAGGCCCTG
CACAACCACTACACCCAGAAGTCTCTGAGCCTGTCTCTGGGCAAG

SEQ ID NO: 30
<223> Amino acid sequence of the heavy chain of Antibody 2
QVQLQQSGSELMMPGASVKISCKATGYTFSNYWIEWVKQRPGHGLEWIGEILPGTGRTIYNE
KFKGKATFTADISSNTVQMQLSSLTSEDSAVYYCARRDYYGNFYYAMDYWGQGTSVTVSSAS
TKGPSVFPLAPCSRSTSESTAALGCLVKDYFPEPVTVSWNSGALTSGVHTFPAVLQSSGLYS
LSSVVTVPSSSLGTKTYTCNVDHKPSNTKVDKRVESKYGPPCPSCPAPEFLGGPSVFLFPPK
PKDTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEEMTKNQVSLTCLVK
GFYPSDIAVEWESNGQPENNYKTTPPVLDSDGSFFLYSRLTVDKSRWQEGNVFSCSVMHEAL
HNHYTQKSLSLSLGK

# Fig. 13

SEQ ID NO: 31
<223>  Nucleotide sequence of the heavy chain of Antibody 3
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGCCACCTTCACCGCCGACATCTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGGCCGCCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

SEQ ID NO: 32
<223>  Amino acid sequence of the heavy chain of Antibody 3
QVQLVQSGAEVKKPGSSVKVSCKASGYTFSNYWIEWVRQAPGQGLEWIGEILPGTGRTIYNE
KFKGRATFTADISTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

# Fig. 14

SEQ ID NO: 33
<223>  Nucleotide sequence of the light chain of Antibodies 3, 4, 5, 6, 10, 11, and 12
GACATCCAGATGACCCAGTCCCCCTCCTCCCTCTCCGCCTCCGTCGGCGACCGCGTCACCAT
CACCTGCTCCGCCTCCCAGGGCATCAACAACTACCTCAACTGGTACCAGCAGAAGCCCGGCA
AGGCCCCCAAGCTCCTCATCTACTACACCTCCACCCTCCAGTCCGGCGTCCCCTCCCGCTTC
TCCGGCTCCGGCTCCGGCACCGACTACACCCTCACCATCTCCTCCCTCCAGCCCGAGGACTT
CGCCACCTACTACTGCCAGCAGTACTCCAAGCTCCCCCGCACCTTCGGCGGCGGCACCAAGG
TCGAGATCAAGCGCACCGTCGCCGCCCCCTCCGTCTTCATCTTCCCCCCCTCCGACGAGCAG
CTCAAGTCCGGCACCGCCTCCGTCGTCTGCCTCCTCAACAACTTCTACCCCCGCGAGGCCAA
GGTCCAGTGGAAGGTCGACAACGCCCTCCAGTCCGGCAACTCCCAGGAGTCCGTCACCGAGC
AGGACTCCAAGGACTCCACCTACTCCCTCTCCTCCACCCTCACCCTCTCCAAGGCCGACTAC
GAGAAGCACAAGGTCTACGCCTGCGAGGTCACCCACCAGGGCCTCTCCTCCCCCGTCACCAA
GTCCTTCAACCGCGGCGAGTGC

SEQ ID NO: 34
<223>  Amino acid sequence of the light chain of Antibodies 3, 4, 5, 6, 10, 11, and 12
DIQMTQSPSSLSASVGDRVTITCSASQGINNYLNWYQQKPGKAPKLLIYYTSTLQSGVPSRF
SGSGSGTDYTLTISSLQPEDFATYYCQQYSKLPRTFGGGTKVEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 35
<223>  Nucleotide sequence of the heavy chain of Antibody 4 and Antibody 7
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGTCACCTTCACCGCCGACATCTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGGCCGCCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

# Fig. 15

SEQ ID NO: 36
<223> Amino acid sequence of the heavy chain of Antibody 4 and Antibody 7
QVQLVQSGAEVKKPGSSVKVSCKASGYTFSNYWIEWVRQAPGQGLEWIGEILPGTGRTIYNE
KFKGRVTFTADISTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

SEQ ID NO: 37
<223> Nucleotide sequence of the heavy chain of Antibody 5 and Antibody 8
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGCCACCTTCACCGCCGACGAGTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGGCCGCCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

SEQ ID NO: 38
<223> Amino acid sequence of the heavy chain of Antibody 5 and Antibody 8
QVQLVQSGAEVKKPGSSVKVSCKASGYTFSNYWIEWVRQAPGQGLEWIGEILPGTGRTIYNE
KFKGRATFTADESTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

# Fig. 16

SEQ ID NO: 39
<223>  Nucleotide sequence of the heavy chain of Antibody 6 and Antibody 9
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGTCACCTTCACCGCCGACGAGTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGGCCGCCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

SEQ ID NO: 40
<223>  Amino acid sequence of the heavy chain of Antibody 6 and Antibody 9
QVQLVQSGAEVKKPGSSVKVSCKASGYTFSNYWIEWVRQAPGQGLEWIGEILPGTGRTIYNE
KFKGRVTFTADESTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPEAAGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

# Fig. 17

SEQ ID NO: 41
<223>  Nucleotide sequence of the light chain of Antibodies 7, 8, and 9
GACATCCAGATGACCCAGTCCCCCTCCTCCCTCTCCGCCTCCGTCGGCGACCGCGTCACCAT
CACCTGCTCCGCCTCCCAGGGCATCAACAACTACCTCAACTGGTACCAGCAGAAGCCCGGCA
AGGCCCCCAAGCTCCTCATCTACTACACCTCCACCCTCCAGTCCGGCGTCCCCTCCCGCTTC
TCCGGCTCCGGCTCCGGCACCGACTTCACCCTCACCATCTCCTCCCTCCAGCCCGAGGACTT
CGCCACCTACTACTGCCAGCAGTACTCCAAGCTCCCCCGCACCTTCGGCGGCGGCACCAAGG
TCGAGATCAAGCGCACCGTCGCCGCCCCCTCCGTCTTCATCTTCCCCCCCTCCGACGAGCAG
CTCAAGTCCGGCACCGCCTCCGTCGTCTGCCTCCTCAACAACTTCTACCCCCGCGAGGCCAA
GGTCCAGTGGAAGGTCGACAACGCCCTCCAGTCCGGCAACTCCCAGGAGTCCGTCACCGAGC
AGGACTCCAAGGACTCCACCTACTCCCTCTCCTCCACCCTCACCCTCTCCAAGGCCGACTAC
GAGAAGCACAAGGTCTACGCCTGCGAGGTCACCCACCAGGGCCTCTCCTCCCCCGTCACCAA
GTCCTTCAACCGCGGCGAGTGC

SEQ ID NO: 42
<223>  Amino acid sequence of the light chain of Antibodies 7, 8, and 9
DIQMTQSPSSLSASVGDRVTITCSASQGINNYLNWYQQKPGKAPKLLIYYTSTLQSGVPSRF
SGSGSGTDFTLTISSLQPEDFATYYCQQYSKLPRTFGGGTKVEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 43
<223>  Nucleotide sequence of the heavy chain of Antibody 10
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGCCACCTTCACCGCCGACATCTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGCTCCTCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

# Fig. 18

SEQ ID NO: 44
<223>  Amino acid sequence of the heavy chain of Antibody 10
QVQLVQSGAEVKKPGSSVKVSCKASGYTFSNYWIEWVRQAPGQGLEWIGEILPGTGRTIYNE
KFKGRATFTADISTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

SEQ ID NO: 45
<223>  Nucleotide sequence of the heavy chain of Antibody 11
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGTCACCTTCACCGCCGACATCTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGCTCCTCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

SEQ ID NO: 46
<223>  Amino acid sequence of the heavy chain of Antibody 11
QVQLVQSGAEVKKPGSSVKVSCKASGYTFSNYWIEWVRQAPGQGLEWIGEILPGTGRTIYNE
KFKGRVTFTADISTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

# Fig. 19

SEQ ID NO: 47
<223> Nucleotide sequence of the heavy chain of Antibody 12
CAGGTCCAGCTCGTCCAGTCCGGCGCCGAGGTCAAGAAGCCCGGCTCCTCCGTCAAGGTCTC
CTGCAAGGCCTCCGGCTACACCTTCTCCAACTACTGGATCGAGTGGGTCCGCCAGGCCCCCG
GCCAGGGCCTCGAGTGGATCGGCGAGATCCTCCCCGGCACCGGCCGCACCATCTACAACGAG
AAGTTCAAGGGCCGCGTCACCTTCACCGCCGACGAGTCCACCTCCACCGCCTACATGGAGCT
CTCCTCCCTCCGCTCCGAGGACACCGCCGTCTACTACTGCGCCCGCCGCGACTACTACGGCA
ACTTCTACTACGCCATGGACTACTGGGGCCAGGGCACCCTCGTCACCGTCTCCTCCGCCTCC
ACCAAGGGCCCCTCCGTCTTCCCCCTCGCCCCCTCCTCCAAGTCCACCTCCGGCGGCACCGC
CGCCCTCGGCTGCCTCGTCAAGGACTACTTCCCCGAGCCCGTCACCGTCTCCTGGAACTCCG
GCGCCCTCACCTCCGGCGTCCACACCTTCCCCGCCGTCCTCCAGTCCTCCGGCCTCTACTCC
CTCTCCTCCGTCGTCACCGTCCCCTCCTCCTCCCTCGGCACCCAGACCTACATCTGCAACGT
CAACCACAAGCCCTCCAACACCAAGGTCGACAAGAAGGTCGAGCCCAAGTCCTGCGACAAGA
CCCACACCTGCCCCCCCTGCCCCGCCCCCGAGCTCCTCGGCGGCCCCTCCGTCTTCCTCTTC
CCCCCCAAGCCCAAGGACACCCTCATGATCTCCCGCACCCCCGAGGTCACCTGCGTCGTCGT
CGACGTCTCCCACGAGGACCCCGAGGTCAAGTTCAACTGGTACGTCGACGGCGTCGAGGTCC
ACAACGCCAAGACCAAGCCCCGCGAGGAGCAGTACAACTCCACCTACCGCGTCGTCTCCGTC
CTCACCGTCCTCCACCAGGACTGGCTCAACGGCAAGGAGTACAAGTGCAAGGTCTCCAACAA
GGCCCTCCCCGCCCCCATCGAGAAGACCATCTCCAAGGCCAAGGGCCAGCCCCGCGAGCCCC
AGGTCTACACCCTCCCCCCCTCCCGCGAGGAGATGACCAAGAACCAGGTCTCCCTCACCTGC
CTCGTCAAGGGCTTCTACCCCTCCGACATCGCCGTCGAGTGGGAGTCCAACGGCCAGCCCGA
GAACAACTACAAGACCACCCCCCCCGTCCTCGACTCCGACGGCTCCTTCTTCCTCTACTCCA
AGCTCACCGTCGACAAGTCCCGCTGGCAGCAGGGCAACGTCTTCTCCTGCTCCGTCATGCAC
GAGGCCCTCCACAACCACTACACCCAGAAGTCCCTCTCCCTCTCCCCCGGC

SEQ ID NO: 48
<223> Amino acid sequence of the heavy chain of Antibody 12
QVQLVQSGAEVKKPGSSVKVSCKASGYTFSNYWIEWVRQAPGQGLEWIGEILPGTGRTIYNE
KFKGRVTFTADESTSTAYMELSSLRSEDTAVYYCARRDYYGNFYYAMDYWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRWQQGNVFSCSVMH
EALHNHYTQKSLSLSPG

Fig. 20

SEQ ID NO: 49
<223> Amino acid sequence of human CD138 protein
MRRAALWLWLCALALSLQPALPQIVATNLPPEDQDGSGDDSDNFSGSGAGALQDITLSQQTP
STWKDTQLLTAIPTSPEPTGLEATAASTSTLPAGEGPKEGEAVVLPEVEPGLTAREQEATPR
PRETTQLPTTHLASTTTATTAQEPATSHPHRDMQPGHHETSTPAGPSQADLHTPHTEDGGPS
ATERAAEDGASSQLPAAEGSGEQDFTFETSGENTAVVAVEPDRRNQSPVDQGATGASQGLLD
RKEVLGGVIAGGLVGLIFAVCLVGFMLYRMKKKDEGSYSLEEPKQANGGAYQKPTKQEEFYA

SEQ ID NO: 50
<223> Specific epitope sequence in CD138
LPEV

**INTERNATIONAL SEARCH REPORT**

International application No. **PCT/JP2024/007871**

**A. CLASSIFICATION OF SUBJECT MATTER**

***A61K 47/68***(2017.01)i; ***A61K 38/08***(2019.01)i; ***A61K 39/395***(2006.01)i; ***A61P 35/00***(2006.01)i; ***A61P 43/00***(2006.01)i
FI: A61K47/68; A61P35/00; A61P43/00 121; A61K39/395 T ZNA; A61K38/08

According to International Patent Classification (IPC) or to both national classification and IPC

**B. FIELDS SEARCHED**

Minimum documentation searched (classification system followed by classification symbols)

A61K47/68; A61K38/08; A61K39/395; A61P35/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | JP 2015-500822 A (BIOTEST AG) 08 January 2015 (2015-01-08) paragraphs [0029], [0033]-[0034], [0231]-[0233], [0242]-[0245], [0254], [0288]-[0289], [0460] | 1-24 |
| Y | SAFDARI, Y. et al. Antibody humanization methods-a review and update. Biotechnol. Genet. Eng. Rev. 2013, 29(2):175-186 table 1 | 1-24 |
| Y | ELKINS, K. et al. FcRL 5 as a Target of Antibody-Drug Conjugates for the Treatment of Multiple Myeloma. Mol. Cancer. Ther. 2012, 11(10):2222-2232 fig. 4-5 | 1-13, 17-24 |
| A | | 14-16 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 May 2024** | **21 May 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/007871**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/007871**

| Patent document cited in search report | Publication date (day/month/year) | Patent family member(s) | Publication date (day/month/year) |
|---|---|---|---|
| JP 2015-500822 A | 08 January 2015 | US 2014/0010828 A1<br>paragraphs [0032], [0037]-[0038], [0323]-[0326], [0619]<br>WO 2013/083817 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009080830 A
- WO 2020214690 A
- JP 2023032088 A

### Non-patent literature cited in the description


- *Bioconjugate Chem.*, 2010, vol. 21, 5-13
- *Current Opinion in Chemical Biology*, 2010, vol. 14, 529-537
- *Expert Opin. Biol. Ther.*, 2004, vol. 4, 1445-1452
- *Nature Biotechnology*, 2012, vol. 30, 631-637
- *Oncotarget*, 2015, vol. 6 (30), 28693-28715
- *Am. J. Physiol. Cell Physiol.*, 01 July 2022, vol. 323 (1), C29-C45
- *Nature*, 2019, vol. 568 (7752), 410-414
- *J. Hematol. Oncol.*, 2017, vol. 10 (1), 13
- *Clin. Lymphoma Myeloma Leuk.*, 2019, vol. 19 (6), 372-380
- *Methods Mol. Biol.*, 2013, vol. 1045, 275-83